(19) Europäisches Patentamt
European Patent Office
Office européen des brevets

(11) Veröffentlichungsnummer: **0 018 473 B1**

(12) **EUROPÄISCHE PATENTSCHRIFT**

(45) Veröffentlichungstag der Patentschrift: **29.12.82**

(51) Int. Cl.³: **C 07 C 121/45**, C 07 C 121/34, C 07 C 121/75, C 07 D 295/14

(21) Anmeldenummer: **80101031.5**

(22) Anmeldetag: **03.03.80**

(54) **Verfahren zur Herstellung von beta-Alkoxy-acrylnitrilen, 3-Amino-acrylnitrilen und 2-Cyano-vinylestern.**

(30) Priorität: **29.03.79 DE 2912345**
**29.03.79 DE 2912344**
**29.03.79 DE 2912343**
**01.08.79 DE 2931228**

(43) Veröffentlichungstag der Anmeldung:
**12.11.80 Patentblatt 80/23**

(45) Bekanntmachung des Hinweises auf die Patenterteilung:
**29.12.82 Patentblatt 82/52**

(84) Benannte Vertragsstaaten:
**BE CH DE FR GB IT NL**

(73) Patentinhaber: **DYNAMIT NOBEL AKTIENGESELLSCHAFT, Patentabteilung**
**Postfach 1209, D-5210 Troisdorf, Bez. Köln (DE)**

(72) Erfinder: **Peeters, Hermann, Dr., Porzer Strasse 1, D-5216 Niederkassel (DE)**
Erfinder: **Prange, Uwe, Dr., Porzer Strasse 7, D-5216 Niederkassel (DE)**
Erfinder: **Vogt, Wilhelm, Dr., Neuenhöfer Allee 54, D-5000 Köln (DE)**

(56) Entgegenhaltungen:
**EP-A-0 001 760**
**AT-B-148 477**
**DE-A-2 107 990**
**DE-A-2 800 764**
**DE-B-1 493 262**
**DE-B-1 807 634**
**FR-A-2 208 887**
**GB-A-806 235**
**US-A-2 375 185**

**BEILSTEINS HANDBUCH DER ORGANISCHEN CHEMIE, 4. Auflage, 3. Ergänzungswerk, 1961, SPRINGER VERLAG, Berlin—Göttingen—Heidelberg, Seite 684**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von β-Alkoxy-acrylnitrilen, 3-Amino-acrylnitrilen und 2-Cyano-vinylestern

Ein Aspekt der Erfindung ist die Herstellung von β-Alkoxyacrylnitrilen aus Metallsalzen von Hydroxy-acryl-nitrilen durch Umsetzung mit Halogenverbindungen.

Die Herstellung von β-Alkoxyacrylnitrilen nach dem Stand der Technik ist schwierig.

Eine umständliche Synthese von β-Äthoxyacrylnitril (I) über mehrere Stufen wird beschrieben durch Umsetzung des Na-Salzes von β-Hydroxyacrylsäureäthylester mit Äthanol in Gegenwart von HCl zum β,β-Diäthoxypropionsäureäthylester, Herstellung des Amids durch Reaktion mit Ammoniak und weitere Dehydratisierung mit $P_2O_5$, wobei (I) als Nebenprodukt gebildet wird (S.M.McElvain und R.L. Clarke, J. Amer. Chem. Soc., 69, [1947] 2657).

In einer anderen, mehrstufigen Synthese wird Chloracetaldehyd mit Blausäure zu α-Hydroxy-β-chlorpropionitril umgesetzt, das wiederum mit Acetanhydrid zu α-Acetoxy-β-chlor propionitril weiterreagiert. Pyrolyse dieser Verbindung führt zu 33% β-Chloracrylnitril. Dieses reagiert mit Alkalialkoholaten zu β-Alkoxyacrylnitril (F. Scotti und E. J. Frazza, J. Org. Chem., 29, [1964] 1800).

β-Alkoxyacrylnitrile können durch Umsetzung von Isoxazol mit NaOH zum Na-Salz des β-Hydroxyacrylnitrils und Alkyllierung dieser Verbindung in situ mit Diäthylsulfat bzw. Alkylhalogeniden (GB-PS 8 06 235) hergestellt werden. Diese Synthese erfordert mehrere Schritte und kostspielige Chemikalien. In Abwesenheit von alkalischen Beschleunigern ergeben sich geringe Ausbeuten von 37,6% und geringe Selektivität durch hohe Anteile von C-Alkylverbindungen. Der langsame Verlauf der Alkylierung mit Dimethylsulfat (US-PS 23 75 185) ist zudem nachteilig.

Der vorliegenden Erfindung liegt daher die Aufgabe zugrunde β-Alkoxyacrylnitrile in einfacher Weise in hoher Ausbeute durch ein technisch und wirtschaftlich leicht zu realisierendes Verfahren herzustellen.

Die Aufgabe wird erfindungsgemäß dadurch gelöst, daß ein Alkali oder Erdalkalisalz von β-Hydroxyacrylnitrilen der allgemeinen Formel A mit Alkylhalogeniden der allgemeinen Formel B in Gegenwart eines Stabilisator- und Katalysatorsystems zu einem β-Alkoxyacrylnitril der allgemeinen Formel C umgesetzt wird.

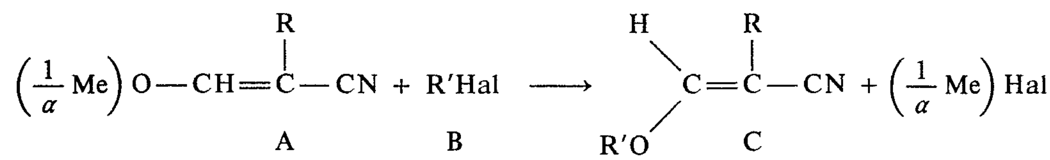

A B C

Gegenstand der Erfindung ist ein Verfahren zur Herstellung von β-Alkoxyacrylnitrilen der Formel

$$R'O-CH=CR-CN \qquad (C)$$

worin R die Bedeutung H, geradkettige oder verzweigte Alkylreste mit 1 bis 20 C-Atomen, geradkettige oder verzweigte Reste $-(CH_2)_n-CN$, $-(CH_2)_n-OR''$ oder $-(CH_2)_n-CH(OR'')_2$ mit $n=0$ bis 5 und $R''$ = Alkylreste mit 1—12 C-Atomen, oder $-(CH_2)_{n+1}-Cyc$, mit Cyc = isocyclische oder heterocyclische, einkernige oder mehrkernige aromatische oder cycloaliphatische Ringsysteme, welche gegebenenfalls Substituenten an den Ringen tragen, hat, und $n=0$ bis 5 ist, und worin R' die Bedeutung geradkettige oder verzweigte Alkyl- oder Alkenylreste mit 1 bis 12 C-Atomen, isocyclische oder heterocyclische, einkernige oder mehrkernige aromatische oder cycloaliphatische Ringsysteme, welche gegebenenfalls Substituenten tragen, oder $-(CH_2)_p-Cyc$ mit Cyc in der vorigen Bedeutung, die Reste $-(CH_2)_p-OR'''$ oder $-(CH_2CH_2-O)_q-R'''$ mit $p=1$ bis 5 und $q=1$ bis 4 und $R'''$ = geradkettige oder verzweigte Alkylreste mit 1 bis 12 C-Atomen, hat, durch Umsetzung von Verbindungen der Formel

$$\left(\frac{1}{\alpha}Me\right)O-CH=CR-CN, \qquad (A)$$

worin R die genannte Bedeutung hat und Me ein Alkalimetall mit $\alpha=1$ oder ein Erdalkalimetall mit $\alpha=2$ ist, mit einer Halogenverbindung der Formel R'—Hal (B), worin R' die obengenannte Bedeutung besitzt, und Hal Chlor, Brom oder Jod bedeutet, bei erhöhter Temperatur, dadurch gekennzeichnet, daß die Reaktion in Gegenwart einer basisch reagierenden Verbindung der Alkali- oder Erdalkalimetalle erfolgt. Die Substituenten R und R' sind allgemein solche, welche sich gegenüber den Reaktionsteilnehmern inert verhalten. Bevorzugt sind in den Substituenten R und R' solche Alkyl- bzw. Alkenylreste mit 1 bis 6 C-Atomen. Unter den cyclische Ringsysteme enthaltenden Resten sind einkernige, d. h. monocyclische stark, von den mehrkernigen die bicyclischen bevorzugt.

In den mehrkernigen Ringsystemen können die Ringe direkt über ein oder mehrere Atome

miteinander oder über ein oder mehrere Kohlenstoff- oder Heteroatome als Brücke miteinander verbunden sein.

Die cycloaliphatischen Ringsysteme sind bevorzugt Cycloalkan-Reste, jedoch können auch eine oder mehrere Doppelbindungen enthalten sein.

Die heterocyclischen Reste enthalten bevorzugt Stickstoff, gegebenenfalls auch Sauerstoff oder weitere Heteroatome. Die gegebenenfalls vorhandenen Substituenten der Ringsysteme sind an sich beliebig, soweit diese bei der Reaktion inert sind. Bevorzugt sind niedere Alkylgruppen von 1 bis 3 C-Atomen, Chlor- Methoxy, Äthoxy- oder Carbalkoxygruppen.

Die zur Herstellung der $\beta$-Alkoxyacrylnitrile verwendeten Alkali- oder Erdalkalisalze von $\beta$-Hydroxyacrylnitrilen der allgemeinen Formel A können nach dem Prinzip der Claisen'schen Esterkondensation aus Alkylcyaniden, Ameisensäureestern und Alkali- oder Erdalkalialkoholaten hergestellt werden. Eine bessere Methode zur Herstellung von Verbindungen der allgemeinen Formel A ist jedoch in der Patentanmeldung P 27 53 322.8 beschrieben, bei der Alkylcyanide mit Alkali- oder Erdalkalialkoholaten in Gegenwart von Kohlenmonoxid umgesetzt werden. Es ist möglich, die nach dieser Vorschrift hergestellten Verbindungen der allgemeinen Formel A direkt, ohne weitere Aufarbeitungsstufe, zu verwenden.

Als Alkali- oder Erdalkaliverbindungen der Formel A werden bevorzugt Na- oder K-Salze wegen ihrer leichten Zugänglichkeit eingesetzt. Es ist jedoch auch möglich, Rb-, Cs, Mg oder Ca-Salze zu verwenden.

Als Halogenverbindung der Formel B werden die Chlorverbindungen bevorzugt. Besonders kommen Methylchlorid, Äthylchlorid, Propylchlorid, Isopropylchlorid, n-Butylchlorid, iso-Butylchlorid, sek.-Butylchlorid, tert.-Butylchlorid 2-Methoxyäthylchlorid, Cyclohexylchlorid, Allylchlorid oder Benzylchlorid in Betracht. Die entsprechenden Bromide oder Jodide können ebenfalls eingesetzt werden, bringen jedoch gegenüber den wirtschaftlich interessanteren Chloriden keine Vorteile.

Die Halogenverbindungen werden in stöchiometrischen Mengen oder in einem Überschuß, vorzugsweise in einem Verhältnis von 1 bis 2 Mol Halogenverbindung je Mol Verbindung der allgemeinen Formel A, eingesetzt. Die überschüssige Halogenverbindung kann recyclisiert werden.

Als Stabilisatorkomponente wird dem Reaktionsgemisch eine basisch reagierende Verbindung der Alkalimetalle oder Erdalkalimetalle zugegeben, beispielsweise Alkalihydroxide, -hydrogencarbonate oder -carbonate sowie Erdalkalioxide, -drogencarbonate oder -carbonate, wie NaOH, KOH, $NaHCO_3$, $KHCO_3$, $Na_2CO_3$, $K_2CO_3$, MgO, CaO, $MgCO_3$ und $CaCO_3$.

Bevorzugt wird CaO eingesetzt.

Die basisch reagierende Verbindung wird in einem Verhältnis von 0,05 bis 1 Äquivalent je Mol Verbindung der allgemeinen Formel A eingesetzt.

Sehr bevorzugt werden Katalysatoren verwendet, die die Selektivität und Geschwindigkeit der Reaktion erhöhen und bevorzugt aus quarternären Ammoniumsalzen der Formel

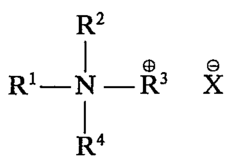

(2)

bestehen, worin $R^1$, $R^2$, $R^3$ und $R^4$ gleiche oder gegebenenfalls verschiedene, lineare oder verzweigte Cycloalkyl, Aryl-, Alkaryl, Aralyl oder Alkylreste mit 1 bis 20 C-Atomen und X ein einwertiges Anion einer Säure bedeuten. Von den cyclischen Resten sind monocyclische Reste, von den aliphatischen Resten die mit 1 bis 6 C-Atomen bevorzugt. Beispiele für $R^1$ bis $R^4$ sind Methyl, Äthyl, Propyl, Butyl, Octyl, Cetyl, Benzyl oder Phenyl. Als Anionen kommen z. B. Fluorid, Chlorid, Bromid, Jodid, Hydrogensulfat, Hexafluorophosphat, Cyanid, Azid, Nitrat, Nitrit, Perchlorat, Tetrafluoroborat, Cyanat, Thiocyanat, Trifluormethansulfonat, 4-Toluensulfonat und Hexafluorarsenat in Betracht. Bevorzugte Verbindung ist Tetra-n-butylammoniumchlorid, -bromid oder -jodid.

Die anstelle der quaternären Ammoniumsalze verwendbaren Kronenäther sind cyclische Äther von Glykolen, in denen die Ziffern die Zahl der Glykolgruppen bezeichnen. In Frage kommen z. B. 15-Krone-5, 18-Krone-6, Dibenzo-18-krone-6 und Dicyclohexyl-18-krone-6.

Das quaternäre Ammoniumsalz der allgemeinen Formel E bzw. der Kronenäther werden in katalytischen Mengen, bezogen auf Verbindung A, eingesetzt. Im allgemeinen wird pro Mol Verbindung A $10^{-3}$ bis $10^{-1}$ Mol quaternäres Ammoniumsalz bzw. Kronenäther eingesetzt. Höhere Konzentrationen sind möglich, jedoch wirtschaftlich unzweckmäßig.

Dem Katalysator kann weiterhin eine Jodverbindung, daher eine aus einem Jodid bestehende oder eine unter Reaktionsbedingungen Jodionen bildende Substanz zugesetzt werden. Hierdurch werden im allgemeinen die höchsten Reaktionsgeschwindigkeiten und besten Ausbeuten erzielt. So kann z. B. das quaternäre Ammoniumsalz der allgemeinen Formel E in Form eines Jodids zugesetzt werden. Weiterhin ist es möglich, dem Alkylhalogenid der allgemeinen Formel B geringe Mengen des entsprechenden Jodids zuzusetzen. Es kann auch ein anorganisches Salz mit einem Jodid-Anion eingesetzt werden. Als Salze können z. B. LiJ, NaJ, KJ, CuJ, $ZnJ_2$ oder $CoJ_2$ verwendet werden.

Die Jodverbindung wird im Verhältnis von $10^{-3}$ bis $10^{-1}$ Mol pro Mol Verbindung der allgemeinen Formel A zugesetzt.

Als Lösungsmittel eignen sich aprotische Lösungsmittel. So können z. B. Alkane, Benzol oder Toluol aus der Reihe der Kohlenwasserstoffe verwendet werden. Äther wie Tetrahydrofuran, Dimethoxyäthan, Di-, Tri- oder Tetraglyme können eingesetzt werden. Polare aprotische Lösungsmittel wie Hexamethylphosphorsäuretriamid, Dimethylsulfoxid, Dimethylformamid oder Acetonitril sind ebenfalls gute Lösungsmittel für die Reaktion.

Das Lösungsmittel kann in Mengen von 0,5—2 l pro Mol Verbindung der allgemeinen Formel A eingesetzt werden. Größere Mengen Lösungsmittel sind möglich, bringen jedoch keine Vorteile.

Als Reaktionstemperatur hat sich ein Bereich von 60 bis 220° C als günstig erwiesen. Besonders bei Einsatz von Chlorverbindungen als Halogenverbindungen der Formel B ist es vorteilhaft, die Reaktionstemperatur im Bereich von 80 bis 180° C zu halten, um genügend schnelle Reaktionsgeschwindigkeiten zu erzielen. Die Reaktionszeit liegt je nach Reaktionstemperatur zwischen 1 bis 8 Stunden, wobei ein vollständiger Umsatz der Verbindung A erreicht wird.

Die Reaktion kann bei Normaldruck, Unterdruck oder Überdruck bis 50 at ausgeführt werden. Höhere Drücke sind möglich, aber nicht zweckmäßig.

Bevorzugt liegt der Druck zwischen Normaldruck und dem Eigendruck der Reaktionsteilnehmer.

Die Reaktion kann wie folgt durchgeführt werden: Der Ausgangsstoff A wird unter Inertgas zusammen mit dem basisch reagierenden anorganischen Salz, dem quaternären Ammoniumsalz und gegebenenfalls der Jod-Verbindung in dem Lösungsmittel suspendiert bzw. gelöst in einem mit einer Rührvorrichtung versehenen Reaktor vorgelegt. Anschließend wird der Ausgangsstoff B zugesetzt, der Reaktor verschlossen und unter Eigendruck auf die Reaktionstemperatur gebracht. Nach Beendigung der Reaktion wird abgekühlt, der Reaktor belüftet und das Reaktionsgemisch entnommen. Durch Filtration oder Zentrifugieren wird der Feststoff abgetrennt und der Endstoff C z. B. durch fraktionierte Destillation isoliert. β-Alkoxyacrylnitrile sind wertvolle Ausgangsstoffe zur Herstellung von heterocyclischen Verbindungen. So läßt sich aus β-Äthoxyacrylnitril und Harnstoff Cytosin herstellen (GB-PS 806 235). Weiterhin lassen sich durch Umsetzung der Endstoffe C mit Hydrazin 3-Amino-4-alkyl-pyrazole, mit Guanidin 2,4-Diamino-5-alkyl-pyrimidine mit Amidinen 2,5-Dialkyl-4-amino-pyrimidine herstellen, die ein breites Anwendungsgebiet auf dem Pharma- und Farbstoffsektor besitzen.

## Allgemeine Versuchsbeschreibung

In einem Reaktor mit Rührvorrichtung werden bei Raumtemperatur unter $N_2$-Atmosphäre ein Alkali- oder Erdalkalisalz eines β-Hydroxyacrylnitrils, ein basisch reagierendes Salz, ein quaternäres Ammoniumsalz der allgemeinen Formel D und eine Jod-Verbindung in einem aprotischen Lösungsmittel vorgelegt. Je nach Aggregatzustand des Alkylhalogenids der allgemeinen Formel B wird dieses ebenfalls zugegeben oder einkondensiert. Der Reaktor wird verschlossen und unter Eigendruck auf die Reaktionstemperatur erhitzt. Nach Beendigung der Reaktion wird abgekühlt und im Falle von unter Normalbedingungen gasförmigen Alkylhalogeniden B der Reaktor entspannt. Das Reaktionsgemisch wird über einen Filter oder eine Zentrifuge von festen Bestandteilen befreit und anschließend fraktioniert destilliert. Je nach Siedepunkt des verwendeten Lösungsmittels wird zuerst dieses oder das Reaktionsprodukt gewonnen. Das Lösungsmittel kann erneut für die Reaktion benutzt werden.

Das Reaktionsgemisch besteht aus einem E-, Z-Isomerengemisch. Bei Einsatz von Verbindungen der allgemeinen Formel A, bei denen R=H ist, enthält das Reaktionsgemisch zusätzlich zum β-Alkoxyacrylnitril C die α-alkylsubstituierte Verbindung D. Um eine bessere destillative Trennung der Hauptprodukte von diesem Nebenprodukt zu erreichen, ist eine Behandlung der Reaktionslösung mit Mineralsäuren wie $H_2SO_4$, $H_3PO_5$ oder HCl vorteilhaft, wodurch jeweils das Z-konfigurierte Isomere in das stabilere E-Isomere übergeführt wird. Dadurch werden größere Siedepunktdifferenzen zwischen dem Hauptprodukt C und dem α-alkylsubstituierten Nebenprodukt (3) erreicht.

$$R'O-CH=\overset{\overset{R'}{\|}}{C}-CN \qquad (3)$$

Ein weiterer Aspekt der Erfindung ist die Herstellung von 3-Aminoacrylnitrilen aus β-Alkoxyacrylnitrilen durch Umsetzung mit Aminen.

Nach dem Stand der Technik waren 3-Aminoacrylnitrile der Formel (1) schwer oder nur über kostspielige Ausgangsprodukte zugänglich, z. B. durch Umsetzung von 3-Chloracrylnitril mit verschiedenen Aminen (J. org. Chem. 29, (1964), 1800), Dehydrierung von 3-Dimethylaminopropionitril. Es war zwar möglich, nach der DE-$A_1$-2 800 764, der DE-B-1 493 262 und der DE-$B_2$-1 807 634 Alkoxymethylenmalodinitril mit aromatischen Aminen umzusetzen, jedoch war hier vorauszusetzen, daß die beiden Nitrilgruppen am selben Kohlenstoffatom einen Sonderfall darstellen, der diesen

Reaktionsweg begünstigt.

Gegenstand der Erfindung ist ein Verfahren zur Herstellung von 3-Aminoacrylnitrilen der Formel (D)

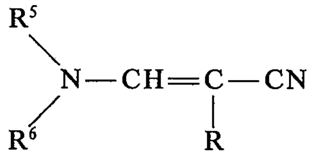

worin $R^5$ und $R^6$ die Bedeutung H, gleiche oder verschiedene, geradkettige oder verzweigte Alkyl-, Alkenyl- oder Alkinyl-Reste mit 1 bis 12 C-Atomen, $-Cyc-(CH_2)_n-Cyc$, worin Cyc isocyclische oder heterocyclische, einkernige oder mehrkernige, aromatische oder cycloaliphatische Ringsysteme, welche gegebenenfalls Substituenten an den Ringen tragen können und $n=0$ bis 5 ist, die Gruppierung $-X-R^7$, worin X geradkettige, verzweigte oder cyclische Alkylen- oder Alkenylreste mit 2 bis 12 C-Atomen, $-Cyc-$, $-(CH_2)_n-Cyc-$ oder $-(CH_2)_n-Cyc-(CH_2)_n-$ mit Cyc und n in der vorigen Bedeutung und $R^7=$

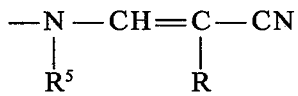

oder $R^5$ und $R^6$ zusammen Alkylen- oder Alkenylen-Reste eines Ringes mit 3 bis 6 Gliedern bilden, der gegebenenfalls durch ein oder mehrere Heteroatome unterbrochen ist, und R die Bedeutung H, geradkettige, verzweigte oder cyclische Alkylreste mit 1 bis 20 C-Atomen, geradkettige oder verzweigte Reste $-(CH_2)_{n+1}-CN$, $-(CH_2)_n-OR''$, $-(CH_2)_n-CH(OR'')_2$ mit $R''=$Alkylreste mit 1 bis 12 C-Atomen oder $-(CH_2)_{n+1}-Cyc$, worin $-Cyc$ und n die obengenannte Bedeutung haben, wobei aber im Falle des Restes $-(CH_2)_{n+1}-CN$, n nicht 5 sein darf, dadurch gekennzeichnet, daß ein 3-Alkoxyacrylnitril der Formel

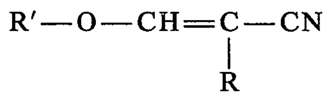

oder ein Metallsalz von 3-Hydroxyacrylnitril mit Ammoniak oder Aminen der Formel

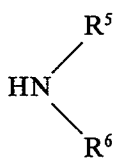

(E)

worin $R^5$ und $R^6$ die obengenannte Bedeutung und R' und R die Bedeutung von R' und R in der Formel (C) haben, umgesetzt werden.

Die Art der Substituenten $R^5$, $R^6$ und R hat sich besonders im Falle der Verwendung der Verfahrensprodukte als Zwischenprodukte für weitere Synthesen nach dem im Endprodukt benötigten Substituenten zu richten. Im allgemeinen sollen sich die Substituenten gegenüber den Reaktionsteilnehmern inert verhalten.

In den mehrkernigen Ringsystemen können die Ringe direkt über ein oder mehrere Atome miteinander oder über ein oder mehrere Kohlenstoff- oder Heteroatome als Brücke miteinander verbunden sein.

Die cycloaliphatischen Ringsysteme sind bevorzugt Cycloalkan-Reste, jedoch können auch eine oder mehrere Doppelbindungen enthalten sein.

Die heterocyclischen Reste enthalten bevorzugt Stickstoff, gegebenenfalls auch Sauerstoff oder weitere Heteroatome. Die gegebenenfalls vorhandenen Substituenten der Ringsysteme sind an sich beliebig, soweit diese bei der Reaktion inert sind. Bevorzugt sind niedere Alkylgruppen von 1 bis 3 C-Atomen, Chlor sowie gegebenenfalls Alkoxyreste.

Als Reste R, soweit diese Alkyl- bzw. Alkenylreste bedeuten, sind kurzkettige mit 1 bis 6 C-Atomen bevorzugt. Unter den cyclische Ringsysteme enthaltenden Resten R sind einkernige, d. h. monocyclische sehr bevorzugt, von den mehrkernigen die bicyclischen bevorzugt.

Als Reste $R^5$ und $R^6$ sind H, niedere aliphatische Reste mit 1—6 C-Atomen, einkernige aromatische oder cycloaromatische Reste wie die des Anilin oder Pyridin, sowie soweit $R^5$ und $R^6$ zusammen Ringe bilden, die Ringe des Morpholins, Pyperidins oder Pyrrolidins bevorzugt. Im Falle der Reaktionsprodukte von Diaminen mit der Gruppierung $R^5=-X-R^7$ ist $R^6$ bevorzugt H.

Als Reste $R^5$ sind besonders die einfach zugängigen niederen Alkylreste mit 1 bis 6 C-Atomen, und von diesen insbesondere Methyl- bis Propylreste bevorzugt.

Das erfindungsgemäße Verfahren wird zweckmäßigerweise so durchgeführt, daß ein 3-Alkoxyacrylnitril und ein Amin ohne Lösungsmittel, in einem inerten polaren organischen Lösungsmittel, z. B. einem Alkohol, Acetonitril, einem polar aprotischen Lösungsmittel, z. B. Dimethylformamid oder Dimethylsulfoxid etc., in einem unpolaren organischen Lösungsmittel, z. B. Benzol, Äther, n- oder cyclo-Paraffin, in Wasser oder geeigneten Lösungsmittelgemischen hiervon umgesetzt wird. Die Menge des eventuell verwendeten Lösungsmittels ist nicht kritisch, zu hohe Verdünnungen verlängern jedoch die Reaktionszeit.

Die Reaktion kann im Temperaturbereich von −30 bis etwa 250° C, vorzugsweise von +10° C bis zum Siedepunkt der Reaktionsteilnehmer, durchgeführt werden. Teilweise werden schon bei niedrigen Temperaturen in kurzer Zeit hohe Ausbeuten erhalten. Die Reaktionszeiten sind abhängig von der angewandten Temperatur und liegen im allgemeinen bei etwa 0,5 bis 5 Stunden.

Die Reaktion kann bei Normaldruck, Unterdruck oder Überdruck bis 50 bar ausgeführt werden. Höhere Drücke sind möglich, aber nicht zweckmäßig. Bevorzugt liegt der Druck zwischen Normaldruck und dem Eigendruck der Reaktionsteilnehmer.

Die Umsetzung kann gegebenenfalls unter Abdestillieren des im Verlauf der Reaktion entstehenden Alkohols R'OH erfolgen, doch ergeben sich dadurch nur insofern Vorteile, als die Reaktionstemperatur z. B. beim Arbeiten unter Normaldruck gegebenenfalls erhöht werden kann.

Das Verhältnis der Moläquivalente von Amin (E) zum 3-Alkoxyacrylnitril (C) kann größer, kleiner oder gleich 1 sein, je nachdem ob eine weitgehend vollständige Umsetzung von Amin (E) oder 3-Alkoxyacrylnitril (C) erreicht werden soll; im allgemeinen wird ein Verhältnis von 4 : 1 bis 1 : 4 eingehalten, jedoch können auch hohe Überschüsse eines Reaktionspartners bis 1 : 10 zweckmäßig sein.

Die Aufarbeitung der Reaktion ist einfach durch Destillation, Kristallisation oder Extraktion mit einem geeigneten Lösungsmittel möglich. Die Reaktionsprodukte fallen mit hoher Ausbeute und hoher Reinheit an.

Die Reaktionsprodukte fallen meistens als cis-/trans-Isomerengemisch an, wobei im allgemeinen das trans.-Isomere überwiegt.

Soweit solche Amine umgesetzt werden sollen, die weniger leicht oder nicht vollständig mit 3-Alkoxyacrylnitrilen der Formel (2) reagieren, beispielsweise den weniger basischen aromatischen Aminen, kann ein zweites reaktionsfähiges Amin zugegeben werden, das die Reaktion katalysiert und im Endprodukt nicht auftritt.

Beispiel für diese reaktionsfähigen Amine ist $NH_3$, jedoch sind auch weitere Amine verwendbar, z. B. Morpholin oder Picolin.

Die Menge des reaktionsfähigen Amins kann ein Äquivalent oder mehr bzw. weniger als ein Äquivalent sein.

Es ist jedoch auch möglich, zunächst die Reaktion mit einem rekationsfähigen Amin durchzuführen, gegebenenfalls auch das Produkt zu isolieren, und danach mit einem der weniger leicht reagierenden Amine das erstrebte Aminoacrylnitril herzustellen.

3-Aminoacrylnitrile der Formel (D) können Verwendung finden zur Darstellung verschiedener Heterocyclen z. B. Cyto- sind, Pyrimidinen, Isoxazolen, 5-Aminopyrazolen, von Malonsäuredinitril, Aminomethylenmalonsäurenitril, von Herbiziden oder als Comonomere zur Herstellung von Polymerisationskunststoffen.

Ein weiterer Aspekt der Erfindung ist die Herstellung von 3-Amino-acrylnitrilen aus Metallsalzen von Hydroxy-acrylnitrilen durch Umsetzung mit Aminen.

Die vorliegende Erfindung betrifft ein Verfahren zur Herstellung von 3-Aminoacrylnitrilen der Formel

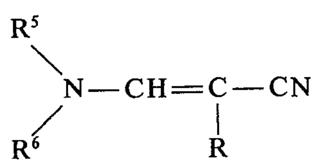

(D)

worin $R^5$, $R^6$ und R die bereits für Stoffe der Formel D genannte Bedeutung haben, welches dadurch gekennzeichnet ist, daß eine Verbindung der Formel

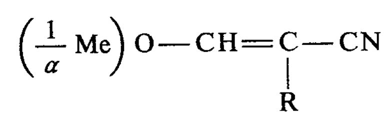

(A)

worin R die obengenannte Bedeutung hat und Me ein Alkalimetall mit $\alpha = 1$ oder ein Erdalkalimetall mit $\alpha = 2$ ist mit einem Amin der Formel

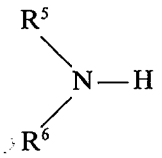

(E)

worin $R^5$ und $R^6$ die für Stoffe der Formel E genannte Bedeutung haben, in Gegenwart einer einbasischen oder mehrbasischen anorganischen oder organischen Säure oder in Gegenwart eines Salzes eines inerten Amins oder mit einem Aminsalz aus einer der genannten Säuren und einem Amin der Formel (E) umgesetzt wird.

Das erfindungsgemäße Verfahren kann so durchgeführt werden, daß die Metallsalze von 3-Hydroxyacrylnitrilen der Formel (A) als Feststoff, Suspension oder gelöst zu der Suspension oder Lösung des Amins der Formel (E) und der einbasischen oder mehrbasischen anorganischen oder organischen Säure oder zu der Suspension oder Lösung des Aminsalzes oder zu der Suspension oder Lösung des Amins der Formel (E) und eines Salzes eines inerten Amins zugegeben wird. Es können auch die Stoffe der Formel (A) und das Amin der Formel (E) gelöst oder suspendiert vorgelegt und darauf die Säure zugegeben werden, oder die Stoffe der Formel (A) vorgelegt und das Aminsalz zugegeben werden. Als inerte Amine werden tertiäre Amine zugesetzt, die wegen der fehlenden Wasserstoffatome nicht an der Reaktion teilnehmen.

Die Ausgangsstoffe sind einfach nach der deutschen Patentanmeldung P 2 753 322.8 zugängig.

Das aus dem Amin der Formel (E) und der Säure zu bildende Aminsalz kann »in situ« in der Reaktionslösung aus dem entsprechenden Amin und der Säure durch Umsetzung im Verhältnis ihrer Moläquivalente hergestellt oder als isoliertes Salz eingesetzt werden. Geeignete Säuren sind zum Beispiel: Salzsäure, Schwefelsäure, Phosphorsäure, Essigsäure, jedoch sind auch weitere gebräuchliche Säuren möglich. Aminsalze sind vorzugsweise die Hydrochloride oder Sulfate. Das Verhältnis der Moläquivalente der Amine bzw. Aminsalze zum Stoff der Formel (A) sollte 1 : 1 bis 6 : 1, vorteilhaft 1 : 1 bis 3 : 1 sein.

Die Menge der Säure bzw. des Salzes eines inerten Amins kann äquivalent zum Amin der Formel (E) sein, jedoch ist auch ein Überschuß von Säure möglich.

Die Umsetzung kann in einem inerten polaren oder unpolaren, neutralen, basischen oder sauren organischen Lösungsmittel, in Wasser oder in geeigneten Gemischen hiervon erfolgen.

Geeignete Lösungsmittel sind zum Beispiel: Alkohole mit 1 bis 6 C-Atomen, Acetonitril, Dimethylformamid, Dimethylsulfoxid, Amine, z. B. ein Überschuß des zur Reaktion gebrachten Amins oder ein inertes tertiäres Amin, Essigsäure, Benzol, Toluol, Äther, doch soll diese Auswahl keine Beschränkung bedeuten.

Die Menge des verwendeten Lösungsmittels wird nach unten bestimmt durch die gute Rührbarkeit der Reaktionsmischung, nach oben sind keine Grenzen gesetzt, doch ergeben zu große Verdünnungen geringere Ausbeuten und eine Verlängerung der Reaktionszeit.

Bei der Umsetzung schwach basischer Amine wie der aromatischen oder heteromatischen Amine ist Wasser als Lösungsmittel oder Bestandteil des Lösungsmittels zu vermeiden. Alle Reaktionsteilnehmer sollen dann im wesentlichen frei von Wasser in getrockneter Form verwendet werden.

Die Reaktion kann im Temperaturbereich von −10° C bis 200° C oder darüber durchgeführt werden, vorzugsweise von 0° C bis zum Siedepunkt des Lösungsmittels. Die Reaktionszeit ist abhängig von der angewandten Reaktionstemperatur und liegt etwa zwischen 1 h und 48 h. Der Druck soll im allgemeinen zwischen Normaldruck und 50 bar liegen. Höhere Drücke sind möglich, aber nicht sinnvoll.

Bevorzugt wird zwischen Normaldruck und dem Eigendruck der Reaktionsteilnehmer gearbeitet.

Die Aufarbeitung des Reaktionsansatzes erfolgt, gegebenenfalls nach Abdestillieren von nicht reagierten und/oder in der Reaktion gebildeten Salz in der Kälte durch Einengen der Mutterlauge und anschließende Destillation oder Kristallisation oder bzw. durch Extraktion mit einem geeigneten Lösungsmittel aus der wäßrigen Reaktionslösung.

Die Reaktionsprodukte fallen im allgemeinen als cis-/trans-Isomerengemisch an, wobei meistens das trans-Isomere überwiegt.

Die Produkte wurden durch IR, $^1$H-NMR und Massenspektroskopie eindeutig charakterisiert.

Der letzte Aspekt der Erfindung ist die Herstellung von 2-Cyano-vinylestern aus Metallsalzen von Hydroxy-acrylnitrilen durch Umsetzung mit Säurehalogeniden oder Säureanhydriden.

Die vorliegende Erfindung betrifft ein Verfahren zur Herstellung von 2-Cyanovinylestern der allgemeinen Formel

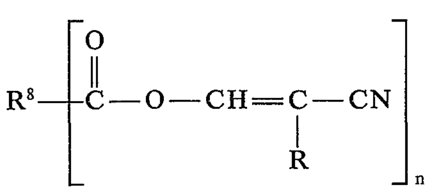

(F)

worin n 1 oder 2 ist und $R^8$ geradkettige oder verzweigte Alkyl-, Alkylen-, Alkenyl- oder Alkenylenreste mit 1 bis 12 C-Atomen, isocyclische oder heterocyclische Ringsysteme mit ein- oder mehrcyclischer Struktur oder $(CH_2)_m$-Cyc, wobei -Cyc ein isocyclischer oder heterocyclischer Ring mit ein- oder mehrcyclischer Struktur, der gegebenenfalls Substituenten am Ring tragen kann und $m = 0$ bis 5 bedeutet, und R die Bedeutung R in der Formel (A) hat, welches dadurch gekennzeichnet ist, daß eine Verbindung der Formel

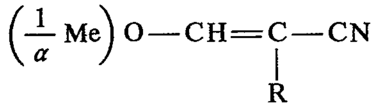

(A)

in der R die für R der Formel A bereits genannte Bedeutung hat und Me ein Alkalimetall mit $\alpha = 1$ oder ein Erdalkalimetall mit $\alpha = 2$ ist, mit einem Säurehalogenid der Formel

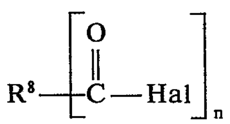

(G)

worin n und $R^8$ die in der Formel (F) angegebene Bedeutung haben und Hal Chlor oder Brom bedeuten, oder einem Säureanhydrid der Formel

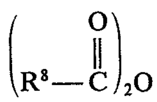

(H)

worin $R^8$ dieselbe Bedeutung wie in der Formel (F) hat, umgesetzt wird.

Nach der US-PS 3 288 779 ist bekannt, 2-Cyanovinylester organischer Carbonsäuren durch Umsetzung von 3-Chloracrylnitril mit Carbonsäuren herzustellen. Aufgrund der schwierigen Darstellung von 3-Chloracrylnitril ist dieses Verfahren technisch unbefriedigend.

Das vorliegende Verfahren stellt einen erheblichen Vorteil gegenüber den bekannten Verfahren dar, da es bei einfacher Reaktionsführung mit hohen Ausbeuten verläuft und die Ausgangsprodukte in Form der Säurechloride (G) oder Säureanhydride (H) sowie die 2-Cyanovinylalkoholate nach der deutschen Patentanmeldung P 2 753 322.8 einfach zugänglich sind.

Die Durchführung des erfindungsgemäßen Verfahrens erfolgt zweckmäßigerweise so, daß das Säurehalogenid der Formel (G) oder das Säureanhydrid der Formel (H), gegebenenfalls gelöst oder suspendiert in einem inerten polaren oder unpolaren Lösungsmittel, zu der Suspension des Cyanovinylalkoholats der Formel (A) in einem polaren oder unpolaren Lösungsmittel zugegeben wird. Bei der Zugabe ist Kühlung auf $-40$ bis 20° C vorteilhaft. Die anschließende Umsetzung erfolgt bei Temperaturen von $-40$ bis 100° C, vorzugsweise $1-10$ bis 50° C.

Geeignete inerte Lösungsmittel sind z. B. Benzol, Toluol, Äther, Tetrahydrofuran, n- oder cyclo-Paraffine, Aceton, Acetonitril, Dimethylformamid oder Dimethylsulfoxid. Die Lösungsmittel müssen wasserfrei sein. Die Menge des verwendeten Lösungsmittels wird nach unten begrenzt durch die Rührbarkeit der Reaktionsmischung, nach oben sind keine Grenzen gesetzt, doch ergeben zu hohe Verdünnungen Ausbeuteminderungen und verlängern die Reaktionszeit. Die Reaktionszeit ist abhängig von der Reaktionstemperatur und der Art der Reaktionsteilnehmer und beträgt im allgemeinen 1 bis 24 Stunden.

Die Ausgangsstoffe können im äquivalenten Mengenverhältnis oder mit einem Überschuß einer Komponente eingesetzt werden, wobei zweckmäßig die leichter zugängige Komponente, d. h. das Säurehalogenid bzw. das Säureanhydrid im Überschuß Verwendung findet.

Das Molverhältnis von Säurehalogenid der Formel (G) oder Säureanhydrid der Formel (H) zu Cyanovinylalkoholat der Formel (A) soll allgemein im Falle $n = 1$ 1 : 1 bis 4 : 1, vorzugsweise 1 : 1 bis 2 : 1, im Falle $n = 2$ 0,5 : 1 bis 2 : 1, vorzugsweise 0,5 : 1 bis 1 : 1 betragen.

Die Aufarbeitung des Reaktionsansatzes erfolgt auf einfache Weise durch Abfiltrieren des gebildeten Salzes Me $Hal_n$ und eventuell nicht umgesetzten Cyanovinylalkoholat, Abdestillieren des Lösungsmittels und überschüssigen Reagenzes und anschließender Destillation oder Kristallisation.

Die 2-Cyanovinylester der Formel (F) können Verwendung finden zur Einführung von 2-Cyanovinylgruppen, zur Darstellung von 2-Cyanoethylcarbonsäureestern durch Hydrieren der Doppelbindung und als Farbstoffe bei Vorliegen eines geeigneten Restes $R^8$.

Es ist zu verstehen, daß an sich die Substituenten $R^8$ und R der verfahrensgemäß hergestellten Stoffe sehr verschiedenartig sind, je nachdem welche Konstitution die als Zwischenprodukte dienenden Cyanovinylester aufweisen sollen und die daraus hergestellten Endprodukte entsprechend dem Verwendungszweck aufzuweisen haben. Demgemäß kommen sowohl einfach aufgebaute

Substituenten $R^8$ und R wie auch speziell aufgebaute Substituenten ganz bestimmter Struktur und mit ganz bestimmten funktionellen Gruppen infrage.

Davon ausgehend, sind für eine Anzahl von Fällen solche Substituenten $R^8$ und R bevorzugt, die den aliphatischen Monocarbonsäuren mit 1 bis 6 C-Atomen, den aliphatischen Dicarbonsäuren mit 1 bis 6 C-Atomen, den unsubstituierten aromatischen Mono- und Dicarbonsäuren sowie deren einfachen Substitutionsprodukten, beispielsweise mit Methyl- oder Chlorsubstituenten, entsprechen.

Bezüglich der Substituenten R sind ebenfalls für zahlreiche Fälle aliphatische Substituenten mit 1 bis 8 C-Atomen oder substituierte oder unsubstituierte Benzylreste bevorzugt.

Die folgenden Beispiele dienen der weiteren Erläuterung der Erfindung.

Die Produkte wurden durch IR, $^1$H-NMR und Massenspektroskopie charakterisiert.

## Beispiele 1 bis 9

In dieser Versuchsreihe wurden verschiedene Alkylierungsmittel R'—Hal eingesetzt.

Es wurden jeweils 0,5 mol NaOCH=CH—CN, 500 ml Acetonitril, 1 g NaJ, 5 g Tetra-n-butylammoniumbromid und 0,5 ml Calciumoxid verwendet.

| Beispiel Nr. | R'-Hal | Reaktions-zeit | Temp. | Ausbeute | |
|---|---|---|---|---|---|
| | | (h) | (°C) | % C | % D |
| 1 | $CH_3Cl$ | 1,5 | 130 | 60 | 15 |
| 2 | $C_2H_5Cl$ | 6 | 130 | 82 | 10 |
| 3 | n-$C_3H_7Br$ | 2,5 | 80 | 75,5 | 12 |
| 4 | iso-$C_3H_7Cl$ | 4 | 175 | 88,9 | 5,2 |
| 5 | iso-$C_3H_7Br$ | 3,5 | 80 | 84,6 | 6 |
| 6 | sek.-$C_4H_9Cl$ | 6 | 150 | 63,04 | 4,5 |
| 7 | Benzylchlorid | 4 | 80 | 50,0 | — |
| 8 | Allylchlorid | 6 | 80 | 55 | *) |
| 9 | Allylbromid | 4 | 80 | 60 | *) |

*) nicht bestimmt.

## Beispiel 10 bis 13

In dieser Versuchsreihe wurden unterschiedliche Ausgangsstoffe A eingesetzt.

Es wurden jeweils 0,5 mol A, 1 mol Äthylchlorid, 500 ml Acetonitril, 1 g NaJ, 5 g Tetra-n-butylammoniumbromid und 0,5 mol CaO verwendet.

| Beispiel Nr. | Verbindung A | Reaktions-zeit, h | Temp., °C | Ausbeute | |
|---|---|---|---|---|---|
| | | | | % C | % D |
| 2 | NaO—CH=CH—CN | 6 | 130 | 82 | 10 |
| 10 | KOCH=CH—CN | 2 | 150 | 84 | 6,5 |
| 11 | 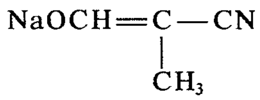 | 2 | 160 | 82 | – |
| 12 | 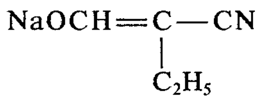 | 2 | 160 | 80 | – |
| 13 | 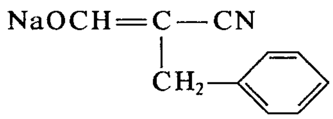 | 6 | 150 | 89,2 | – |

## Beispiel 14 bis 19

In dieser Versuchsreihe wurden verschiedene aprotische Lösungsmittel verwendet.

Es wurde jeweils 0,5 mol A, 1 mol $C_2H_5Cl$, 500 ml Lösungsmittel, 1 g NaJ, 5 g Tetra-n-butylammoniumbromid und 0,5 mol CaO eingesetzt.

A = NaOCH = CH–CN

| Beispiel Nr. | Lösungsmittel | Reaktions-zeit | Temp. | Ausbeute | |
|---|---|---|---|---|---|
| | | (h) | (°C) | % C | % D |
| 14 | Acetonitril | 2 | 160 | 83 | 12 |
| 15 | Aceton*) | 2 | 120 | 78 | 10 |
| 16 | Benzol | 4 | 160 | 84 | 12 |
| 17 | Essigsäureäthylester | 2 | 130 | 73 | 10 |
| 18 | 1,2-Dimethyloxyäthan | 2 | 130 | 60 | 11,6 |
| 19 | Dimethylformamid | 2 | 130 | 80 | 11,4 |

*) zusätzliche Bildung von Diacetonalkohol.

## Beispiel 20 bis 22

In dieser Versuchsreihe wurden verschiedene Jodid-Promotoren untersucht.

Es wurden jeweils 0,5 mol NaO–CH=CH–CN, 1 mol $C_2H_5$–Cl, 500 ml Acetonitril, 1 g Jodid-Promotor, 5 g Tetra-n-butylammoniumbromid und 0,5 mol CaO eingesetzt.

| Beispiel Nr. | Jodid-Promotor | Reaktions-zeit | Temp. | Ausbeute | |
|---|---|---|---|---|---|
| | | (h) | (°C) | % C | % D |
| 14 | NaJ | 2 | 160 | 83 | 12 |
| 20 | KJ | 2 | 160 | 82 | 12 |
| 21 | $ZnJ_2$ | 2 | 160 | 80 | 11 |
| 22 | $C_2H_5J$ | 2 | 150 | 82 | 14 |

Beispiel 23 bis 26

In dieser Vesuchsreihe wurden verschiedene basische Verbindungen als Stabilisatoren verwendet. Es wurden jeweils 0,5 mol NaOCH=CH—CN, 1 mol $C_2H_5Cl$, 500 ml Lösungsmittel, 1 g NaJ, 5 g Tetra-n-butylammoniumbromid und 0,5 mol basische Verbindung eingesetzt. Die Reaktionszeit betrug 2 h bei 160°C.

| Beispiel Nr. | bas. Komponente | Lösungsmittel | Ausbeute | |
|---|---|---|---|---|
| | | | % C | % D |
| 14 | CaO | Acetonitril | 83 | 12 |
| 23 | MgO | Benzol | 68 | 14 |
| 24 | NaOH | Benzol | 60 | 20 |
| 25 | $NaOOCCH_3$ | Acetonitril | 49 | 10 |
| 26 | $K_2CO_3$ | Acetonitril | 62 | 11 |

Beispiel 27 bis 30

In dieser Versuchsreihe wurden verschiedene quaternäre Ammoniumsalze E oder Kronenäther verwendet.
Es wurden jeweils 0,5 mol NaOCH=CH—CN, 1 mol $C_2H_5$—Cl, 500 ml Acetonitril, 1 g NaJ, 0,5 mol CaO eingesetzt. Die Reaktionszeit betrug 2 Stunden bei 160°C.

| Beispiel Nr. | D oder Kronenäther | Menge | Ausbeute | |
|---|---|---|---|---|
| | | g | % C | % D |
| 14 | Tetra-n-butylammoniumbromid | 5 | 83 | 12 |
| 27 | Tetra-n-butylammoniumbromid | 2,5 | 82 | 11 |
| 28 | Tetra-n-butylammoniumchlorid | 3 | 82 | 12 |
| 29 | Dibenzo-18-krone-6 | 5 | 59 | 13 |
| 30 | 18-Krone-6 | 2,5 | 69,4 | 15,2 |

Beispiele zur Herstellung von 3-Amino-acrylnitrilen. Die hergestellten Substanzen wurden durch $^1H$—NMR- sowie IR-Spektren sowie massenspektrometrisch identifiziert.

Beispiel 31

19,4 g (0,2 mol) 3-Ethoxyacrylnitril (=3-EAN) und 25,5 g (1,5 mol) Ammoniak werden in einem Autoklaven 4 h auf 100°C erhitzt. Nach Ablassen des Ammoniaks und Abdestillieren des Äthanols werden 11,0 g (81%) 3-Aminoacrylnitril erhalten.

$^{1}H-NMR$ ($CDCl_3$):
$\delta$=3.96 (d.$J_{cis}$=9.2 Hz, CH—CN), 4.25 (d, $J_{tr}$=14 Hz, CH—CN), 4.8—6.4 (breit, NH), 6.4—7.9 (m, CH—N).

Beispiel 32

19,4 g (0,2 mol) 3-EAN und 38 g 40% wäßrige Methylaminlösung (0,5 mol) werden 3 h bei 25°C gerührt. Nach Extraktion mit Äther, Trocknen und Destillation im Vakuum werden 15,4 g (93,8% bezogen auf 3-EAN) 3-Methylaminoacrylnitril erhalten.

$^{1}H-NMR$ ($CDCl_3$):
2.68 Cd, $J_{NH}$=5.0 Hz, $CH_{3tr}$, 2.8), 2.98 (d, $J_{NH}$=5,0 Hz, $CH_{3\,cis}$, 0,2), 3.67 (d, $J_{cis}$=8,5 Hz, CH—CN), 3.83 (d, $J_{tr}$=13,5 Hz, CH—CN), 5.71 (breit, NH, 1), 6.60 (dd, $J_{NH}$=12,0 Hz, $J_{cis}$=8,5 Hz, CH—N), 7.12 (dd, $J_{NH}$=7.1 Hz, $J_{tr}$=13,5 Hz, CH—N).

Beispiel 33

48,5 g (0,5 mol) 3-EAN und 90 g (2 mol) Dimethylamin in 150 ml Benzol werden 3 h bei 55°C gehalten. Anschließende Destillation im Vakuum ergibt 46,1 g (96,1% bezogen auf 3-EAN) 3-Dimethylaminoacrylnitril.

$^{1}H-NMR$ ($CCl_4$):
$\delta$=2,92 (s, $CH_{3tr}$, 5,4), 3,17 (s, $CH_{3\,cis}$, 0,6), 3,57 (d, $J_{cis}$=9,5 Hz, CH—CN, 0,1), 3,75 (d, $J_{tr}$=13,5 Hz, CH—CN, 0,9), 6,50 (d, $J_{cis}$=9,5 Hz, CH—N, 0,1), 7,15 (d, $J_{tr}$=13,5 Hz, CH—N, 0,9).

Beispiel 34

9,7 g (0,1 mol) 3-EAN und 21,9 g (0,3 mol) Diäthylamin werden 4 h zum Rückfluß erhitzt. Anschließende Destillation im Vakuum ergibt 9,9 g (80% bezogen auf 3-EAN) 3-Diäthylaminoacrylnitril.

$^{1}H-NMR$ ($CCl_4$):
$\delta$=1,14 (t, J=7,0 Hz, $CH_{3tr}$, 5,8), 1,28 (t, J=7,0 Hz, $CH_{3cis}$, 0,2), 3,18 (q, J=7,0 Hz, $CH_{2tr}$), 3,38 (q, J=7,0 Hz, $CH_{2cis}$), 3,71 (d, $J_{tr}$=13,5 Hz, CH—CN, 1), 6,31 (d, $J_{cis}$=9,0 Hz, CH—N), 6,90 (d, $J_{tr}$=13,5 Hz, CH—N, 1).

Beispiel 35

14,6 g (0,15 mol) 3-EAN und 24,8 g (0,25 mol) Cyclohexylamin werden unter Abdestillieren von Ethanol 2 h zum Rückfluß erhitzt. Nach dem Abkühlen wird der Feststoff abgesaugt und mit kaltem Methanol gewaschen. Ausbeute 21,4 g (95,1% bezogen auf 3-EAN) 3-Cyclohexylaminoacrylnitril.

$^{1}H-NMR$ ($CdCl_3$):
$\delta$=1—2 (m, $(CH_2)_5$, 10), 3.03 (m, 1), 3.75 (d, $J_{cis}$=8,4 Hz, CH—CN, 0,34), 3.93 (d, $J_{tr}$=13.8 Hz, CH—CN, 0,66), 4,88 (m, NH, 1) 6,66 (dd, $J_{NH}$=13,2 Hz, $J_{cis}$=8.4 Hz, CH—N), 6.95 (dd, $J_{NH}$=9.0 Hz, $J_{tr}$ 13.8 Hz, (CH—N).

Beispiel 36

19,4 g (0,2 mol) 3-EAN und 21,3 g (0,3 mol) Pyrrolidin werden 2 h zum Rückfluß erhitzt. Die anschließende Destillation im Vakuum ergibt 23,5 g (96,3% bezogen auf 3-EAN) 3-Pyrrolidinacrylnitril.

$^{1}H-NMR$ ($CDCl_3$):
$\delta$=1.95 (m, $(CH_2)_2$, 4), 3.23 (m, $(CH_2)_2$, 4), 3.62 (d, $J_{tr}$=13,3 Hz, CH—CN, 1), 6.53 (d, $J_{cis}$=9.0 Hz, CH—N, 0,05), 7.14 (d, $J_{tr}$=13,3 Hz, CH—N, 0,95).

Beispiel 37

9,7 g (0,1 mol) 3-EAN und 21,4 g (0,2 mol) Benzylamin werden unter Abdestillieren von Ethanol 4 h zum Rückfluß erhitzt. Anschließende Destillation im Vakuum ergibt 11,4 g (72% bezogen auf 3-EAN) 3-Benzylaminoacrylnitril.

$^1H-NMR$ ($CDCl_3$):
$\delta = 3.88$ (d, $J_{tr} = 13.8$ Hz, CH—CN, 1) 4.12 (d, $J_{NH} = 5.5$ Hz, $CH_2$, 2), 5.3—6.2 (breit, NH) 6.7—7.6 (m, Ph, CH—N, 6).

Beispiel 38

19,4 g (0,2 mol) 3-EAN und 17 g (0,3 mol) Allylamin werden 3 h zum Rückfluß erhitzt. Anschließende Destillation im Vakuum ergibt 20,3 g (94% bezogen auf 3-EAN) 3-Allylaminoacrylnitril.

$^1H-NMR$ ($CDCl_3$):
$\delta = 3.52-3.98$ (m, $CH_{2aliph.}$, CH—CN, 3), 5.09—5.32 (m, $CH_{2olef.}$), 5.66—5.86 (m, CH—C), 5.7 (breit, NH), 6.63 (dd, $J_{NH} = 12,7$ Hz, $J_{cis} = 8,3$ Hz, CH—N, 0,24), 7.05 (dd, $J_{NH} = 8.0$ Hz, $J_{tr} = 13,8$ Hz, CH—N, 0,76).

Beispiel 39

14,55 g (0,15 mol) 3-EAN und 4,5 g (0,075 mol) Ethylendiamin werden 5 h unter Abdestillieren von Ethanol zum Rückfluß erhitzt. Nach Filtration und Waschen mit kaltem Methanol werden 10,7 g (88%) 3,3-(N,N'-Ethylendiamino-)bisacrylnitril erhalten.

$^1H-NMR$ (Aceton · $d_6$):
$\delta = 3.16-3.55$ (m, $CH_2$, 4) 3.72 (d, $J_{cis} = 8,4$ Hz, CH—CN, 0,34) 4.06 (d, $J_{tr} = 13,8$ Hz, CH—CN, 1.66), 6.30 (s, NH, 2) 6.78 (dd, $J_{NH} = 12,7$ Hz, $J_{cis} = 8.4$ Hz, CH—N) 7.12 (dd, $J_{NH} = 8.1$ Hz, $J_{tr} = 13,8$ Hz, CH—N).

Beispiel 40

9,7 g (0,1 mol) 3-EAN und 12,8 g (0,15 mol) Piperidin werden 1,5 h zum Rückfluß erhitzt. Anschließende Destillation im Vakuum ergibt 12,5 g (91,7% bezogen auf 3-EAN) 3-Piperidinoacrylnitril.

$^1H-NMR$ ($CCl_4$):
$\delta = 1,58$ (m), $(CH_2)_3$,6), 3.13 (m, $(CH_2)_2$, 4) 3.81 (d, $J_{tr} = 13.5$ Hz, CH—CN), 6.37 (d, $J_{cis} = 9.0$ Hz, CH—N, 0.3), 6.93 (d, $J_{tr} = 13,5$ Hz, CH—N, 0,7).

Beispiel 41

16,7 g (0,15 mol) 3-n-Propoxyacrylnitril und 17,0 g (0,2 mol) Piperidin werden 5 h zum Rückfluß erhitzt. Durch Destillation werden 17,1 g (84% bezogen auf n-Propoxyacrylnitril) 3-Piperidinoacrylnitril erhalten.
Das Spektrum ist gleich dem Spektrum in Beispiel 10.

Beispiel 42

48,5 g (0,5 mol) 3-EAN und 21,8 g (0,25 mol) Morpholin werden 5 h zum Rückfluß erhitzt. Anschließende Destillation im Vakuum ergibt 31,3 g (90,7% bezogen auf Morpholin) 3-Morpholinoacrylnitril.

$^1H-NMR$ ($CDCl_3$):
$\delta = 3.11-3.75$ (m, $(CH_2)_4$, CH—$CN_{cis}$), 3.94 (d, $J_{tr} = 13,7$ Hz, CH—CN), 6.30 (d, $J_{cis} = 9.7$ Hz, CH—N, 0,46), 6.90 (d, $J_{tr} = 13,7$ Hz, CH—N, 0,54).

Beispiel 43

19,4 g (0,2 mol) 3-EAN und 8,6 g (0,1 mol) Piperazin werden 2 h zum Rückfluß erhitzt. Der nach dem Abkühlen anfallende Feststoff wird abgesaugt und mit kaltem Methan gewaschen. Es werden 12,6 g (67%) 3,3'-Piperazinobisacrylnitril erhalten.

$^1H-NMR$ ($DMSO-D_6$):
$\delta = 3{,}24$ (s, $CH_2$, 8) 4.17 (d, $J_{tr} = 13.5$ Hz, CH—CN, 2) 7.20 (d, $J_{tr} = 13{,}5$ Hz, CH—N, 2).

Beispiel 44

19,4 g (0,2 mol) 3-EAN, 13.6 g (0,8 mol) Ammoniak und 14,0 g (0,15 mol) Anilin werden 4 h in einem Autoklaven auf 100°C erhitzt. Anschließend wird in Wasser aufgenommen und mit Äther extrahiert. Die Umkristallisation des eingeengten Extraktes mit Tetrachlorkohlenstoff ergibt 19.2 g (88.9% bezogen auf Anilin) 3-Anilinoacrylnitril.

$^1H-NMR$ ($DMSO-D_6$):
$\delta = 4.67$ (d, $J_{tr} = 13.8$ Hz, CH—CN, 1), 6.9—7.7 (m, Ph), 7.76 (dd, $J_{NH} = 12.8$ Hz, $J_{tr} = 13.8$ Hz, CH—N), 9.6 (d, breit, NH).

Beispiel 45

19,4 g (0,2 mol) 3-EAN, 13,6 g (0,8 mol) Ammoniak und 21,6 g (0,2 mol) 2-Aminopicolin werden 4 h in einem Autoklaven auf 100°C erhitzt, anschließend wird in Wasser aufgenommen und mit Äther extrahiert. Umkristallisation des eingeengten Extraktes mit Tetrachlorkohlenstoff ergibt 24,2 g (76%) 2-($\beta$-Cyanovinyl-)amino-6-methylpyridin.

$^1H-NMR$ (DMSO $d_6$):
$\delta = 2.38$ (s, $CH_3$, 3), 4.42 (d, $J_{cis} = 9.0$ Hz, CH—CN, 0,96), 4.84 (d, $J_{tr} = 14{,}0$ Hz, CH—CN, 0,04), 6.8—7.6 (m, $CH_{arom.}$), 8.00 (dd, $J_{NH} = 12{,}5$ Hz, $J_{cis} = 9.0$ Hz, CH—N), 9.96 (d, breit, J = 12.5 Hz, NH, 1).

Beispiel 46

11,1 g (0,1 mol) 2-Methyl-3-ethoxyacrylnitril und 15,2 g (0,2 mol) 40% wäßrige Methylaminlösung werden 15 h bei 25°C gehalten. Nach Extraktion mit Äther, Trocknen der Ätherphase und Destillation im Vakuum werden 8.15 g (84,9% bezgl. 2-Methyl-3-ethoxyacrylnitril) 2-Methyl-3-methylaminoacrylnitril erhalten.

$^1H-NMR$ ($CDCl_3$):
$\delta = 1{,}63$ (d, J = 1,2 Hz, $CH_3-C_{tr}$, 2,6), 1.73 (d, J = 1.0 $CH_3-C_{cis}$, 0,4), 2.92 Cd, $J_{NH} = 4.8$ Hz, $CH_3-N$, 3), 4.30 (s, breit, NH, 1), 6.44 (dq, $J_{NH} = 12.5$ Hz, J = 1.0 Hz, $CH_{cis}$), 6.63 (dq, $J_{NH} = 13.3$ Hz, J = 1.2 Hz, $CH_{tr}$).

Beispiel 47

2-Benzyl-3-ethoxyacrylnitril wird mit Piperidin mit guter Ausbeute analog dem Beispiel 40 zum 2-Benzyl-3-piperdinoacrylnitril umgesetzt.

Beispiel 48

9,35 g (0,05 mol) 2-Benzyl-3-ethoxyacrylnitril und 17,4 g (0,2 mol) Morpholin werden 20 h zum Rückfluß erhitzt. Anschließende Destillation im Vakuum ergibt 4,6 g (81% bezogen auf umgesetztes 2-Benzyl-3-ethoxyacrylnitril (50%) 2-Benzyl-3-morpholinacrylnitril.

$^1H$-NMR ($CDCl_3$):
$\delta = 3.2-3.8$ (m, $CH_2$, 8), 6.32 (s, CH, 1), 7.28 (s, Ph, 5).

Die folgenden Beispiele 49 bis 66 betreffen die Herstellung von 3-Amino-acrylnitrilen aus Metallsalzen von 3-Hydroxyacrylnitril:

Beispiel 49

Zu einer Suspension von 32.5 g (0,25 mol) Natrium-3-hydroxyacrylnitril (Gehalt 70%) in 200 ml mit Ammoniak gesättigtem Ethanol werden unter Kühlung langsam 12—35 g (0,126 mol) Schwefelsäure in 40 ml Ethanol gegeben und 5 h bei 60°C gerührt. Nach Abfiltrieren vom Feststoff, Abziehen des Lösungsmittels, Extraktion des Rückstandes mit Essigester und Abziehen des Extraktionsmittels wurden 13.1 g (77%) 3-Aminoacrylnitril erhalten.

$^1H-NMR$ ($CDCl_3$):
$\delta$=3.96 (d, $J_{cis}$=9.2 Hz, CH—CN), 4.25 (d, $J_{tr}$=13.8 Hz, CH—CN), 4.8—6.4 (breit, NH), 6.4—7.9 (m, CH—N).

Beispiel 50

32.5 g (0,25 mol) Natrium-3-hydroxiacrylnitril (Gehalt 70%) und 20,3 g (0,3 mol) Methylaminhydrochlorid werden in 100 ml Ethanol 4 h zum Rückfluß erhitzt. Nach Abfiltrieren vom Feststoff, Abziehen des Lösungsmittels und anschließender Destillation im Vakuum wurden 15,3 g (74,5%) 3-Methylaminoacrylnitril erhalten.

$^1N-NMR$ ($CDCl_3$):
$\delta$=2.68 (d, $J_{NH}$=5.0 Hz, $CH_{3tr}$, 2.8), 2.98 (d, $J_{NH}$=5.0 Hz, $CH_{3cis}$, 0,2), 3.67 (d, $J_{cis}$=8.5 Hz, CH—CN), 3.83 (d, $J_{tr}$=13.5 Hz, CH—CN), 5.71 (breit, NH, 1) 6.60 (dd, $J_{cis}$=12.0 Hz, $J_{cis}$=8.5 Hz, CH—CN), 7.12 (dd, $J_{NH}$=7.1 Hz, $J_{tr}$=13,5 Hz, CH—CN).

Beispiel 51

32,5 g (0,25 mol) Natrium-3-Hydroxiacrylnitril (Gehalt 70%) und 24,3 g (0,3 mol) Dimethylaminhydrochlorid werden in 100 ml Ethanol 40 h auf 50°C erhitzt. Nach Aufarbeitung wie in Beispiel 50 wurden 16.8 (70%) 3-Dimethylamino-acrylnitril erhalten.

$^1H-NMR$ ($CCl_4$):
$\delta$=2.92 (s, $CH_{3tr}$, 5.8), 3.17 (s, $CH_{3cis}$, 0,2), 3.57 (d, $J_{cis}$=9.5 Hz, CH—CN), 3.75 (d, $J_{tr}$=13,5 Hz, CH—CN), 6,50 (d, $J_{cis}$=9.5 Hz, CH—N, 0,1), 17,15 (d, $J_{tr}$=13,5 Hz, CH—N, 0,9).

Beispiel 52 (Stoff des Beispiels 51)

30,3 g (0,25 mol) Natrium-3-hydroxiacrylnitril (Gehalt 75%) und 24,3 g (0,3 mol) Dimethylaminhydrochlorid werden in 200 ml Wasser 2 h bei 20°C gehalten. Nach Extraktion mit Chloroform und Destillation im Vakuum werden 20,1 g (83,7%) 3-Dimethylaminoacrylnitril erhalten.

Das $^1H-NMR$ Spektrum ist das gleiche wie im Beispiel 51.

Beispiel 53

32,5 g (0,25 mol) Natrium-3-hydroxiacrylnitril (Gehalt 70%) werden zu einer Lösung von 25,6 g (0,35 mol Diäthylamin, 21,0 g (0,35 mol) Essigsäure und 150 ml Acetonitril gegeben und 16 h bei 25°C gerührt. Nach Aufarbeitung wie im Beispiel 50 wurden 19.7 g (63,6%) 3-Diäthylaminoacrylnitril erhalten.

$^1H-NMR$ ($CCl_4$):
$\delta$=1.14 (t, J=7.0 Hz, $CH_{3tr}$, 5.8), 1.28 (t, J=7.0 Hz, $CH_{3cis}$, 0,2), 3.18 (q, J=7.0 Hz, $CH_{2tr}$), 3.38 (q, J=7.0 Hz, $CH_{2cis}$), 3.71 (d, $J_{tr}$=13,5 Hz, CH—CN), 6.31 (d, $J_{cis}$=9.0 Hz, CH—N), 6.90 (d, $J_{tr}$=13,5 Hz, CH—N, 1).

Beispiel 54

32,5 g (0,25 mol) Natrium-3-hydroxiacrylnitril (Gehalt 70%) werden zu einer Suspension von Cyclohexylaminhydrochlorid (1aus 29,8 g [0,3 mol] Cyclohexylamin und gasförmiger Salzsäure) in 150 ml Ethanol gegeben und 24 h bei 25°C gerührt. Nach Abfiltrieren vom Feststoff, Abziehen des Lösungsmittels und anschließender Umkristallisation aus Tetrachlorkohlenstoff wurden 32,1 g (85,6%) 3-Cyclohexylaminoacrylnitril erhalten.

$^1H-NMR$ ($CDCl_3$):
$\delta$ = 1—2 (m, $(CH_2)_5$, 10), 3.03 (m, 1), 3.75 (d, $J_{cis}$ = 8.4 Hz, CH—CN, 0,04), 3.93 (d, $J_{tr}$ = 13,8 Hz, CH—CN, 0,96), m, NH, 1), 6.66 (dd, $J_{NH}$ = 13,2 Hz, $J_{cis}$ = 8.4 Hz, CH—CN) 6.95 (dd, $J_{NH}$ = 9.0 Hz, $J_{tr}$ = 13.8 Hz, CH—N).

Beispiel 55

32,5 g (0,25 mol) Natrium-3-hydroxiacrylnitril (Gehalt 70%) werden zu einer Mischung aus 17,8 g (0,25 mol) Pyrrolidin, 12,3 g (0,125 mol) Schwefelsäure und 200 ml Ethanol zugegeben und 7 h bei 50°C gerührt. Nach Aufarbeitung wie in Beispiel 50 wurden 24,3 g (79,6%) 3-Pyrrolidinacrylnitril erhalten.

$^1H-NMR$ ($CDCl_3$):
$\delta$ = 1.95 (m, $(CH_2)_2$4), 3.23 (m, $(CH_2)_2$, 4), 3.62 (d, $J_{tr}$ = 13,3 Hz, CH—CN, 1), 6.53 (d, $J_{cis}$ = 9.0 Hz, CH—N, 0,04), 7.14 (d, $J_{tr}$ = 13,3 Hz, CH—N, 0,96).

Beispiel 56

32,5 g (0,25 mol) Natrium-3-hydroxiacrylnitril (Gehalt 70%) und 43,1 g (0,3 mol) Benzylaminhydrochlorid werden in 150 ml Acetonitril 5 h zum Rückfluß erhitzt. Nach dem Abfiltrieren vom Feststoff und Abziehen des Lösungsmittels wurde in Chloroform aufgenommen und mit Wasser gewaschen. Die anschließende Destillation im Vakuum ergab 22,6 g (57,1%) 3-Benzylaminoacrylnitril.

$^1H-NMR$ ($CDCl_3$):
$\delta$ = 3.88 (d, $J_{tr}$ = 13.8 Hz, CH—CN, 1), 4.12 (d, $J_{NH}$ = 5.5 Hz, $CH_2$, 2), 5.3—6.2 (breit, NH), 6.7—7.6 (m, Ph, CH—N, 6).

Beispiel 57

Zu der Suspension von 30,3 g (0,25 mol) Natrium-3-hydroxiacrylnitril (Gehalt 75%) und 17 g (0,3 mol) Allylamin in 150 ml Methanol werden bei 10°C 18 g (0,3 mol) Essigsäure zugegeben und 48 h bei 25°C gerührt. Nach Aufarbeitung wie in Beispiel 50 wurden 19,2 g (71,1%) 3-Allylaminoacrylnitril erhalten.

$^1H-NMR$ ($CDCl_3$):
$\delta$ = 3.52—3.98 (m, $CH_{2aliph.}$, CH—CN, 3), 5.09—5.32 (m, $CH_{2olef.}$), 5.66—5.86 (m, CH—C), 5.7 (breit, NH), 6.63 (dd, $J_{NH}$ = 12.7 Hz, $J_{cis}$ = 8.3 Hz, CH—N, 0,2), 7.05 (dd, $J_{NH}$ = 8,0 Hz, $J_{tr}$ = 13.8 Hz, CH—N, 0,8).

Beispiel 58

30,3 g (0,25 mol) Natrium-3-hydroxiacrylnitril (Gehalt 75%) werden zu einer Lösung von 9 g (0,15 mol) Ethylendiamin in 150 ml 2 n Salzsäure gegeben und 24 h bei 25°C gerührt. Der ausgefallene Feststoff wird abfiltriert und getrocknet, die Mutterlauge mit Chloroform extrahiert. Es werden 12,1 g (59,8%) 3,3-(N,N-Ethylendiamino-)bisacrylnitril erhalten.

$^1H-NMR$ (Aceton.$d_6$):
$\delta$ 3.16—3.55 (m, $CH_2$, 4), 3.72 (d, $J_{cis}$ = 8,4 Hz, CH—CN, 0,3), 4.06 (d, $J_{tr}$ = 13,8 Hz, CH—CN, 1.66), 6.30 (s, NH, 2), 6.78 (dd, $J_{NH}$ = 12.7 Hz, $J_{cis}$ = 8,4 Hz, CH—N), 7.12 (dd, $J_{NH}$ = 8.1 Hz, $J_{tr}$ = 13.8 Hz, CH—N).

Beispiel 59

30,3 g (0,25 mol, Natrium-3-hydroxiacrylnitril (Gehalt 75%) werden zu einer Suspension von Piperidinsulfat (aus 29,8 g [0,35 mol] Piperidin und 17,15 g [0,175 mol] Schwefelsäure) in 200 ml Methanol gegeben und 24 h bei 25°C gerührt. Nach Aufarbeitung wie in Beispiel 55 wurden 28,2 g (82,9%) 3-Piperidinoacrylnitril erhalten.

$^1H-NMR$ ($CCl_4$):
$\delta$ = 1.58 (m, $(CH_2)_3$, 6), 3.13 (m, $(CH_2)_2$, 4), 3.81 (d, $J_{tr}$ = 13.5 Hz, CH—CN), 6.37 (d, $J_{cis}$ = 9,0 Hz, CH—N, 0,1), 6.93 (d, $J_{tr}$ = 13,5 Hz, CH—N, 0,9).

Beispiel 60 (Stoff des Beispiels 59)

30,3 g (0,25 mol) Natrium-3-hydroxiacrylnitril (Gehalt 75%) werden zu einer Suspension von 41,3 g (0,3 mol) Triäthylaminhydrochlorid und (21,3 g [0,25 mol] Piperidin) in 200 ml Ethanol gegeben und 24 h bei 25° C gehalten. Nach Aufarbeitung wie im Beispiel 56 wurden 26,9 g (79,3%) 3-Piperidinoacrylnitril erhalten.

Das $^1H-NMR$-Spektrum ist das gleiche wie im Beispiel 59.

Beispiel 61

30,3 g (0,25 mol) Natrium-3-hydroxiacrylnitril (Gehalt 75%) werden zu einer Lösung von Morpholinacetat (aus 26,1 g [0,3 mol] Morpholin und 18 g [0,3 mol] Essigsäure) in 200 ml Acetonitril gegeben und 8 h auf 65°C erhitzt. Nach Aufarbeitung wie im Beispiel 55 wurden 26,0 g (75,4%) 3-Morpholinacrylnitril erhalten.

$^1H-NMR$ ($CDCl_3$):
$\delta$=3.11—3.75 (m, $(CH_2)_4$, CH—$CN_{cis}$), 3.94 (d, $J_{tr}$=13.7 Hz, CH—CN), 6.30 (d, $J_{cis}$=9.7 Hz, CH—N, 0,2), 6.90 (d, $J_{tr}$=13,7 Hz, CH—N, 0,8).

Beispiel 62

30,3 g (0,25 mol) Natrium-3-hydroxiacrylnitril (Gehalt 75%) werden zu einer Suspension von Piperazinsulfat (aus 12,9 g [0,15 mol] Piperazin und 14,7 g [0,15 mol] Schwefelsäure) in 200 ml Acetonitril gegeben und 5 h zum Rückfluß erhitzt. Nach Abfiltrieren vom Feststoff und Abziehen des Lösungsmittels wurde in Chloroform aufgenommen und mit Wasser gewaschen. Nach Abziehen des Chloroforms und Umkristallisation des Rückstandes aus Essigester/Tetrachlorkohlenstoff wurden 12,5 g (53,2%) 3,3-Piperazino-bisacrylnitril erhalten.

$^1H-NMR$ (DMSO—$D_6$):
$\delta$=3.24 (s, $CH_2$, 8), 4.17 (d, $J_{tr}$=13.5 Hz, CH—CN, 2), 7.20 (d, $J_{tr}$=13.5 Hz, CH—N, 2).

Beispiel 63

Zu einer Suspension von 32,5 g (0,25 mol) Natrium-3-hydroxiacrylnitril und 32,6 g (0,35 mol) Anilin in 150 ml Ethanol werden bei 0°C 12,25 g (0,125 mol) Schwefelsäure in 30 ml Ethanol zugetropft und 5 h bei 25°C gehalten. Nach dem Abfiltrieren von Feststoff und Abziehen des Lösungsmittels wird im Wasser aufgenommen, mit verdünnter Schwefelsäure angesäuert und mit Äther extrahiert. Nach Umkristallisation des eingeengten Ätherextraktes aus Tetrachlorkohlenstoff wurden 24,3 g (67,6%) 3-Anilinoacrylnitril erhalten.

$^1H-NMR$ (DMSO—$d_6$):
$\delta$=4.67 (d, $J_{tr}$=13.8 Hz, CH—CN, 1), 6.9—7.7, (m, Ph), 7.76 (dd, $J_{NH}$=12.8 Hz, $J_{tr}$=13.8 Hz, CH—N), 9.6, (d, breit, NH).

Beispiel 64

Zu einer Suspension vin 32,5 g (0,25 mol) Natrium-3-hydroxiacrylnitril und 37,8 g (0,35 mol) 2-Aminopicolin in 150 ml Ethanol werden bei 0°C 12,25 g (0,125 mol) Schwefelsäure in 30 ml Ethanol zugetropft und 5 h bei 60°C gehalten. Nach der Aufarbeitung wie im Beispiel 61 wurden 24,9 g (62,7%) 2-($\beta$-Cyanovinyl-)amino-6-methylpyridin erhalten.

$^1H-NMR$ (DMSO—$d_6$):
$\delta$=2.38 (s, $CH_3$, 3), 4.42 (d, $J_{cis}$=9.0 Hz, CH—CN, 0.98), 4.84 (d, $J_{tr}$=14.0 Hz, CH—CN, 0,02), 6.8—7.6 (m, $CH_{arom.}$), 8.00 (dd, $J_{NH}$=12,5 Hz, $J_{cis}$=9.0 Hz, CH—N), 9.96 (d, breit, J=12.5 Hz, NH, 1).

Beispiel 65

7,35 g (0,1 mol) Natrium-2-methyl-3-hydroxiacrylnitril (Gehalt 70%) und 10,1 g (0,15 mol), Methylaminhydrochlorid werden in 100 ml Ethanol 20 h bei 20°C gerührt. Nach Aufarbeitung wie in Beispiel 50 wurden 6,9 g (72,0%) 2-Methyl-3-methylaminoacrylnitril erhalten.

Beispiel 66

10,9 g (0,05 mol) Natrium-2-benzyl-2-hydroxiacrylnitril (Gehalt 83%) und 12,5 g (0,15 mol) Dimethylaminhydrochlorid werden in 40 ml Wasser 5 h bei 20°C gehalten. Nach Extraktion mit Methylenchlorid und Destillation im Vakuum werden 7,2 g (89%) 2-Benzyl-3-dimethylaminoacrylnitril erhalten.

$^1H-NMR$ ($CDCl_3$):
$\delta$=3.03 (s, $CH_3$, 6), 3.38 (s, $CH_2$, 2) 6,37 (s, CH, l), 7.32 (s, Ph, 5).

Beispiel 67

Zu einer Suspension von 25,6 g (0,2 mol) Natrium-3-hydroxiacrylnitril (Gehalt 71%) in 150 ml Aceton werden bei 0°C langsam unter Rühren 27,6 g (0,35 mol) Acetylchlorid zugegeben. Nach 2 h Reaktion bei 20°C wird das Salz abgetrennt und die Mutterlauge eingeengt. Die anschließende Destillation im Vakuum ergibt 19,5 g (88%) Essigsäure-(2-cyanovinyl)-ester.

$^1H-NMR$ ($CDCl_3$):
$\delta$=2,25 (s, $CH_{3tr}$), 2.32 (s, $CH_{3cis}$), 3 H; 5,10 (d, $J_{cis}$=7 Hz, CH—CN), 5,43 (d, $J_{tr}$=13,4 Hz, CH—CN) l H; 7.93 (d, $J_{cis}$=7 Hz, CH—O, 0,55) 8,17 (d, $J_{tr}$=13,4 Hz, CH—O, 0,45).

Beispiel 68

Beispiel 67 wird wiederholt, jedoch mit Äther statt Aceton als Lösungsmittel.

Ausbeute:
15,7 g (70,8%) Essigsäure-(2-cyanovinyl)-ester.

Beispiel 69

Zu einer Suspension von 25,6 g (0,2 mol) Natrium-3-hydroxiacrylnitril (Gehalt 71%) in 150 ml Benzol werden bei 0°C langsam 22,5 g (0,25 mol) Essigsäureanhydrid zugegeben und 3 h bei 25°C gerührt. Nach Abtrennen des Feststoffes und Abziehen des Lösungsmittels wird im Vakuum destilliert.

Ausbeute:
16,5 g (74,3%) Essigsäure-(2-cyanovinyl)-ester.

Beispiel 70

Zu einer Suspension von 24,3 g (0,2 mol) Natrium-3-hydroxiacrylnitril (Gehalt 75%) in 150 ml Acetonitril werden bei 0°C langsam unter Rühren 22,5 (0,25 mol) Essigsäureanhydrid zugegeben und 2 h bei 20°C gerührt. Nach Abtrennen des Feststoffes un Abziehen des Lösungsmittels wird im Vakuum destilliert.

Ausbeute:
21,6 g (97,3%) Essigsäure-(2-cyanovinyl)-ester.

Beispiel 71

Zu einer Suspension von 25,6 g (0,2 mol) Natrium-3-hydroxiacrylnitril (Gehalt 71%) in 150 ml Äther wird bei 0°C langsam unter Rühren 35,1 g (0,25 mol) Benzoylchlorid zugegeben und 20 h bei 25°C gerührt.
Es wird wie im Beispiel 67 aufgearbeitet.

Ausbeute:
23,6 g (68%) Benzoesäure-(2-cyanovinyl)-ester.
$^1H$-NMR ($CDCl_3$):
$\delta$=5.11 (d, $J_{cis}$=6.8 Hz, CH—CN), 5.51 (d, $J_{tr}$=13 Hz, CH—CN), 7.2—8.6 (m, Ph und CH—O).

## Beispiel 72

Zu einer Suspension von 9,1 g (0,075 mol) Natrium-3-hydroxiacrylnitril (Gehalt 75%) in 75 ml Äther werden bei $-20°C$ 13,1 g (0,075 mol) p-Chlorbenzoylchlorid in 30 ml Äther zugetropft und 1 h bei 0°C gehalten. Nach Abtrennen des Feststoffes und Abziehen des Lösungsmittels wird aus Essigester umkristallisiert.

Ausbeute:
8,9 g (57%) p-Chlorbenzoesäure-(2-cyanvinyl)-ester.
$^1H-NMR$ ($CDCl_3$):
$\delta=5.18$ (d, $J_{cis}=6.8$ Hz, CH—CN), 5.58 (d, $J_{tr}=13,5$ Hz, CH—CN) 1 H; 7.3—8.7 (m, Ph und CH—O, 5).

## Beispiel 73

Zu einer Suspension von 25,6 g (0,2 mol) Natrium-3-hydroxiacrylnitril (Gehalt 71%) in 200 ml Benzol wird bei 10°C langsam unter Rühren 19,4 g (0,125 mol) Bernsteinsäuredichlorid zugegeben und 2 h bei 20°C gerührt. Der nach Abtrennen des Feststoffes und Abziehen des Lösungsmittels erhaltene Rückstand wird mit Tetrachlorkohlenstoff gewaschen und abgesaugt. Als Filtrat verbleiben 4,4 g (20%) Bernsteinsäure-bis-(2-cyanovinyl)-ester.

$^1H-NMR$ ($DMSO-d_6$):
$\delta=2.96$ (s, $CH_2$, 4); 5.47 (d, $J_{cis}=7$ Hz, CH—CN), 5.82 (d, $J_{tr}=13,5$ Hz, CH—CN) 2 H; 8,07 (d, $J_{cis}=7$ Hz, CH—O, 08), 8.30 (d, $J_{tr}=13,5$ Hz, CH—O, 1,2).

## Beispiel 74

Zu einer Suspension von 24,2 g (0,2 mol) Natrium-3-hydroxiacrylnitril (Gehalt 75%) in 200 ml Äther werden bei $-10°C$ 20,2 g (0,1 mol) Isophthalsäuredichlorid in 50 ml Äther zugetropft und 2 h bei 0°C gehalten. Nach Abtrennen des Feststoffes und Abziehen des Lösungsmittels wird aus Essigester umkristallisiert.

Ausbeute:
11,8 g (44%) Isophthalsäure-bis-(2-cyanovinyl)-ester.
$^1H$-NMR ($DMSO-d_6$):
$\delta=5.74$ (d, $J_{cis}=6.8$ Hz, CH—CN), 6.15 (d, $J_{tr}=13$ Hz, CH—CN) 2 H; 7.5—8.7 (m, Ph und CH—O) 6 H.

## Beispiel 75

Zu einer Suspension von 28 g (0,187 mol) Natrium-2-methyl-3-hydroxiacrylnitril (Gehalt 70%) in 125 ml Toluol wird bei $<10°C$ langsam unter Rühren 24,8 g (0,243 mol) Acetanhydrid zugegeben und 24 h bei 20°C gerührt. Es wird wie im Beispiel 67 aufgearbeitet.

Ausbeute:
18,7 g (80%) Essigsäure- (2-Methyl-2-cyanovinyl)-ester.
$^1H-NMR$ ($CDCl_3$):
$\delta=1,92$, 2,00 (s, $CH_3-C-CN_{cis,\,tr}$,3), 2,32 (s, $CH_3-C=0$,3, 7,6—8,1 (m, CH, 1).

## Beispiel 76

Zu einer Suspension von 4,5 g (0.02 mol) Natrium-2-benzyl-3-hydroxiacrylnitril (Gehalt 83%) in 20 ml Acetonitril werden bei 0°C langsam unter Rühren 2,65 g (0,026 mol) Acetanhydrid zugetropft und 3 h bei 20°C gerührt. Nach Abtrennen des Feststoffes und Abziehen des Lösungsmittels wird im Vakuum destilliert.

Ausbeute:
3,44 g (93%) Essigsäure-(2-benzyl-2-cyanovinyl)-ester.
$^1H$-NMR ($CDCl_3$):
$\delta=2,09$ (s, $CH_{3cis}$), 2,12 (s, $CH_{3tr}$), 3 H; 3,43 (d, J=1,0 Hz, $CH_{2cis}$, 0,76) 3,60 (d, J=1,0 Hz, $CH_{2tr}$, 1,24); 7,31 (s, Ph, 5); 7,81 (t, J=1,0 Hz, $CH_{cis}$), 7,92 (t, J=1,0 Hz, $CH_{tr}$) 1 H.

**Patentansprüche**

1. Verfahren zur Herstellung von $\beta$-Alkoxyacrylnitrilen der Formel

$$R'O-CH=CR-CN \qquad (C)$$

worin R die Bedeutung H, geradkettige oder verzweigte Alkylreste mit 1 bis 20 C-Atomen, geradkettige oder verzweigte Reste $-(CH_2)_n-CN$, $-(CH_2)_n-OR''$ oder $-(CH_2)_n-CH(OR'')_2$ mit n = 0 bis 5 und R'' = Alkylreste mit 1 — 12 C-Atomen, oder $-(CH_2)_{n+1}-Cyc$, mit Cyc = isocyclische oder heterocyclische, einkernige oder mehrkernige aromatische oder cycloaliphatische Ringsysteme, welche gegebenenfalls Substituenten an den Ringen tragen, hat, und n = 0 bis 5, und worin R' die Bedeutung geradkettige oder verzweigte Alkyl- oder Alkenylreste mit 1 bis 12 C-Atomen, isocyclische oder heterocyclische, einkernige oder mehrkernige aromatische oder cycloaliphatische Ringsysteme, welche gegebenenfalls Substituenten tragen, oder $-(CH_2)_p-Cyc$ mit Cyc in der vorigen Bedeutung, die Reste $-(CH_2)_p-OR'''$ oder $-(CH_2-CH_2-O)_q-R'''$ mit p = 1 bis 5 und q = 1 bis 4 und R''' = geradkettige oder verzweigte Alkylreste mit 1 bis 12 C-Atomen, hat, durch Umsetzung von Verbindungen der Formel

$$\left(\frac{1}{\alpha}\,Me\right)O-CH=CR-CN\,, \qquad (A)$$

worin R die obengenannte Bedeutung hat und Me ein Alkalimetall mit $\alpha = 1$ oder ein Erdalkalimetall mit $\alpha = 2$ ist, mit einer Halogenverbindung der Formel R'—Hal (B), worin R' die obengenannte Bedeutung besitzt und Hal Chlor, Brom oder Jod bedeutet bei erhöhter Temperatur, dadurch gekennzeichnet, daß die Reaktion in Gegenwart einer basisch reagierenden Verbindung der Alkali- oder Erdalkalimetalle erfolgt.

2. Verfahren nach Anspruch 1, dadurch gekennzeichnet, daß ein quaternäres Ammoniumsalz oder ein Kronenäther als Katalysator zugegeben wird.

3. Verfahren nach Anspruch 2, dadurch gekennzeichnet, daß als Katalysator eine quaternäre Ammoniumbase der allgemeinen Formel

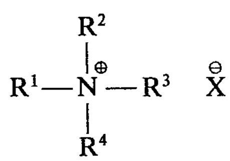
(2)

verwendet wird, worin $R^1$, $R^2$, $R^3$ und $R^4$ gleiche oder verschiedene Cycloalkyl-, Aryl-, Alkaryl-, Aralkyl- oder Alkylreste, die linear oder verzweigt sein können, mit 1—20 C-Atomen und X ein einwertiges Anion bedeuten.

4. Verfahren nach Anspruch 2, dadurch gekennzeichnet, daß als Katalysator ein Kronenäther verwendet wird.

5. Verfahren nach einem der Ansprüche 3 und 4, dadurch gekennzeichnet, daß das quaternäre Ammoniumsalz bzw. der Kronenäther in Mengen von $10^{-3}$ bis $10^{-1}$ Mol, bezogen auf Verbindung A, eingesetzt wird.

6. Verfahren nach einem oder mehreren der Ansprüche 1 bis 5, dadurch gekennzeichnet, daß als weiterer Katalysator eine Jodverbindung in Form eines Jodids oder einer zur Bildung von Jodionen befähigten Verbindung zugesetzt wird.

7. Verfahren nach Anspruch 6, dadurch gekennzeichnet, daß die Jod-Verbindung im Verhältnis von $10^{-3}$ bis $10^{-1}$ Mol pro Mol Verbindung der allgemeinen Formel A zugesetzt wird.

8. Verfahren nach einem oder mehreren der Ansprüche 1 bis 7, dadurch gekennzeichnet, daß als Lösungsmittel ein aprotisches Medium verwendet wird.

9. Verfahren nach einem oder mehreren der Ansprüche 1 bis 8, dadurch gekennzeichnet, daß die Reaktionstemperatur zwischen 60 bis 220, bevorzugt zwischen 80 bis 180° C, liegt.

10. Verfahren zur Herstellung von 3-Aminoacrylnitrilen der Formel

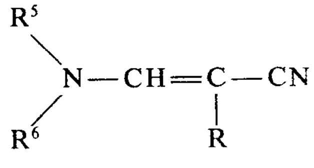
(D)

worin $R^5$ und $R^6$ die Bedeutung H, gleiche oder verschiedene, geradkettige oder verzweigte Alkyl-, Alkenyl- oder Alkinyl-Reste mit 1 bis 12 C-Atomen, —Cyc, $-(CH_2)_n-Cyc$, worin Cyc isocyclische oder

heterocyclische, einkernige oder mehrkernige, aromatische oder cycloaliphatische Ringsysteme, welche gegebenenfalls Substituenten an den Ringen tragen können und n=0 bis 5 ist, die Gruppierung $-X-R^7$, worin X geradkettige, verzweigte oder cyclische Alkylen- oder Alkenylenreste mit 2 bis 12 C-Atomen, —Cyc—, $-(CH_2)_n-$Cyc— oder $-(CH_2)_n-$Cyc$-(CH_2)_n-$ mit Cyc und n in der vorigen Bedeutung und $R^7$=

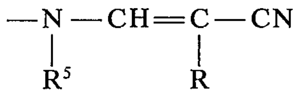

$R^5$ und $R^6$ zusammen Alkylen- oder Alkenylen-Reste eines Ringes mit 3 bis 6 Gliedern bilden, der gegebenenfalls durch ein oder mehrere Heteroatome unterbrochen ist, und R die Bedeutung H, geradkettige, verzweigte oder cyclische Alkylreste mit 1 bis 20 C-Atomen, geradkettige oder verzweigte Reste $-(CH_2)_{n+1}-CN$, $-(CH_2)_n-OR''$, $-(CH_2)_n-CH(OR'')_2$ mit R''=Alkylreste mit 1 bis 12 C-Atomen oder $-(CH_2)_{n+1}-$Cyc, worin —Cyc und n die obengenannte Bedeutung haben, wobei aber im Falle des Restes $-(CH_2)_{n+1}-CN$, n nicht 5 sein darf, dadurch gekennzeichnet, daß ein 3-Alkoxyacrylnitril oder ein Metallsalz von 3-Hydroxyacrylnitrilen mit Ammoniak oder einem primären oder sekundären Amin, gegebenenfalls in Gegenwart einer ein- oder mehrbasischen anorganischen oder organischen Säure, bzw. mit den Ammoniumsalzen dieser Säuren umgesetzt wird.

11. Verfahren zur Herstellung von 3-Aminoacrylnitrilen nach Anspruch 10 der Formel

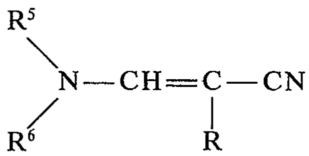

(D)

worin $R^5$ und $R^6$ die Bedeutung H, gleiche oder verschiedene, geradkettige oder verzweigte Alkyl-, Alkenyl- oder Alkinyl-Reste mit 1 bis 12 C-Atomen, —Cyc, $-(CH_2)_n-$Cyc, worin Cyc isocyclische oder heterocyclische, einkernige oder mehrkernige, aromatische oder cycloaliphatische Ringsystem, welche gegebenenfalls Substituenten an den Ringen tragen können und n=0 bis 5 ist, die Gruppierung $-X-R^7$, worin X geradkettige, verzweigte oder cyclische Alkylen- oder Alkenylenreste mit 2 bis 12 C-Atomen, —Cyc—, $-(CH_2)_n-$Cyc— oder $-(CH_2)_n-$Cyc$-(CH_2)_n-$ mit Cyc und n in der vorigen Bedeutung und $R^7$=

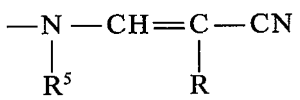

oder $R^5$ und $R^6$ zusammen Alkylen- oder Alkenylen-Reste eines Ringes mit 3 bis 6 Gliedern bilden, der gegebenenfalls durch ein oder mehrere Heteroatome unterbrochen ist, und R die Bedeutung H, geradkettige, verzweigte oder cyclische Alkylreste mit 1 bis 20 C-Atomen, geradkettige oder verzweigte Reste $-(CH_2)_{n+1}-CN$, $-(CH_2)_n-OR''$, $-(CH_2)_n-CH(OR'')_2$ mit R'''=Alkylreste mit 1 bis 12 C-Atomen oder $-(CH_2)_{n+1}-$Cyc, worin —Cyc und n die obengenannte Bedeutung haben, wobei aber im Falle des Restes $-(CH_2)_{n+1}-CN$, n nicht 5 sein darf, dadurch gekennzeichnet, daß ein 3-Alkoxyacrylnitril der Formel

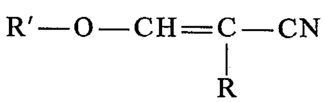

(C)

worin R die obengenannte Bedeutung hat und R' die Bedeutung geradkettige oder verzweigte Alkyl- oder Alkenylreste mit 1 bis 12 C-Atomen, isocyclische oder heterocyclische, einkerniger oder mehrkernige, aromatische oder cycloaliphatische Ringsysteme, welche gegebenenfalls Substituenten tragen, oder $-(CH_2)_p-$Cyc mit Cyc in der vorigen Bedeutung, die Reste $-(CH_2)_p-OR'''$ oder $-(CH_2-CH_2-O)_q-R'''$ mit p=1 bis 5 und q=1 bis 4 und R'''=geradkettige oder verzweigte Alkylreste mit 1 bis 12 C-Atomen, hat, mit Aminen der Formel

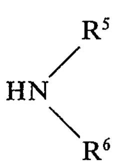

(E)

worin $R^5$ und $R^6$ die obengenannte Bedeutung haben, umgesetzt wird.

12. Verfahren nach Anspruch 11, dadurch gekennzeichnet, daß die Reaktion bei Temperaturen von $-30$ bis 250° C, vorzugsweise von 0° C bis zum Siedepunkt der Reaktionsteilnehmer, durchgeführt wird.

13. Verfahren nach einem der Ansprüche 11 und 12, dadurch gekennzeichnet, daß die Reaktion in Gegenwart eines zusätzlichen Amins durchgeführt wird, das die Reaktion katalysiert.

14. Verfahren zur Herstellung von 3-Aminoacrylnitrilen nach Anspruch 10 der Formel

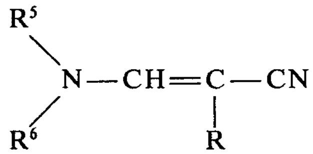

(D)

worin $R^5$ und $R^6$ die Bedeutung H, gleiche oder verschiedene, geradkettige oder verzweigte Alkyl-, Alkenyl- oder Alkinyl-Reste mit 1 bis 12 C-Atomen, $-Cyc$, $-(CH_2)_n-Cyc$, worin Cyc isocyclische oder heterocyclische, einkernige oder mehrkernige, aromatische oder cycloaliphatische Ringsysteme, welche gegebenenfalls Substituenten an den Ringen tragen können und $n=0$ bis 5 ist, die Gruppierung $-X-R^7$, worin X geradkettige, verzweigte oder cyclische Alkylen- oder Alkenylenreste mit 2 bis 12 C-Atomen, $-Cyc-$, $-(CH_2)_n-Cyc-$ oder $-(CH_2)_n-Cyc-(CH_2)_n-$ mit Cyc und n in der vorigen Bedeutung und $R^7=$

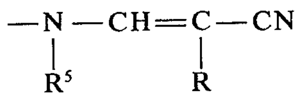

oder $R^5$ und $R^6$ zusammen Alkylen- oder Alkenylen-Reste eines Ringes mit 3 bis 6 Gliedern bilden, der gegebenenfalls durch ein oder mehrere Heteroatome unterbrochen ist, und R die Bedeutung H, geradkettige, verzweigte oder cyclische Alkylreste mit 1 bis 20 C-Atomen, geradkettige oder verzweigte Reste $-(CH_2)_{n+1}-CN$, $-(CH_2)_n-OR'$, $-(CH_2)_n-CH(OR)_2$ mit $R'=$Alkylreste mit 1 bis 12 C-Atomen oder $-(CH_2)_{n+1}-Cyc$, worin $-Cyc$ und n die obengenannte Bedeutung haben, wobei aber im Falle des Restes $-(CH_2)_{n+1}-CN$, n nicht 5 sein darf, dadurch gekennzeichnet, daß eine Verbindung der Formel

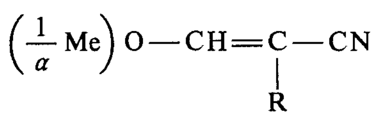

(A)

worin R die obengenannte Bedeutung hat und Me ein Alkalimetall mit $\alpha = 1$ oder ein Erdalkalimetall mit $\alpha = 2$ ist mit einem Amin der Formel

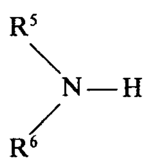

(E)

worin $R^5$ und $R^6$ die obengenannte Bedeutung haben, in Gegenwart einer einbasischen oder mehrbasischen anorganischen oder organischen Säure oder in Gegenwart eines Salzes eines inerten Amins oder mit einem Aminsalz aus einer der genannten Säuren und einem Amin der Formel (E) umgesetzt wird.

15. Verfahren nach Anspruch 14, dadurch gekennzeichnet, daß die Reaktion im Temperaturbereich von $-10$° C bis 200° C, vorzugsweise von 0° C bis zum Siedepunkt der Reaktionsteilnehmer durchgeführt wird.

16. Verfahren nach den Ansprüchen 14 oder 15, dadurch gekennzeichnet, daß das Verhältnis der Moläquivalente des Amins der Formel (E) bzw. des Aminsalzes zum Stoff der Formel (A) 1 : 1 bis 6 : 1, vorzugsweise 1 : 1 bis 3 : 1, beträgt.

17. Verfahren zur Herstellung von 2-Cyanovinylestern der allgemeinen Formel

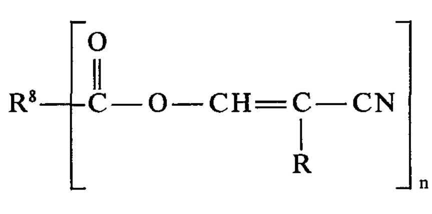

(F)

worin n 1 oder 2 ist und $R^8$ geradkettige oder verzweigte Alkyl-, Alkylen-, Alkenyl- oder Alkenylenreste mit 1 bis 12 C-Atomen, isocyclische oder heterocyclische Ringsysteme mit ein- oder mehrcyclischer Struktur oder $(CH_2)_m-Cyc$, wobei $-Cyc$ ein isocyclischer oder heterocyclischer Ring mit ein- oder mehrcyclischer Struktur, der gegebenenfalls Substituenten am Ring tragen kann und m=0 bis 5 bedeutet und R H, geradkettige, verzweigte oder cyclische Alkylreste mit 1 bis 20 Kohlenstoff-Atomen, geradkettige oder verzweigte Reste $(CH_2)_p-CN$, $(CH_2)_p-COOR'''$, $(CH)_p-NH_2$, $(CH_2)_p-OR'''$ · $(CH_2)_{p+1}-Cyc$, wobei Cyc ein isocyclischer oder heterocyclischer Ring mit ein- oder mehrcyclischer Struktur oder ein aromatischer oder heteroaromatischer Ring mit ein- oder mehrcyclischer Struktur, der gegebenenfalls Substituenten am Ring tragen kann, R''' Alkylreste mit 1 bis 12 C-Atomen oder Reste einwertiger Phenole und p=0 bis 5 ist, bedeutet, dadurch gekennzeichnet, daß eine Verbindung der Formel

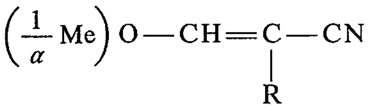 (A)

in der R dieselbe Bedeutung wie in der Formel (F) hat und Me ein Alkalimetall mit $\alpha = 1$ oder ein Erdalkalimetall mit $\alpha = 2$ ist, mit einem Säurehalogenid der Formel

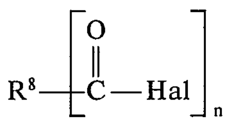 (G)

worin n und $R^1$ die in der Formel (F) angegebene Bedeutung haben und Hal Chlor oder Brom bedeuten, oder einem Säureanhydrid der Formel

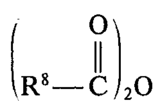 (H)

worin $R^8$ dieselbe Bedeutung wie in der Formel (F) hat, umgesetzt wird.

18. Verfahren nach Anspruch 17, dadurch gekennzeichnet, daß die Umsetzung in einem inerten, polaren oder unpolaren organischen Lösungsmittel erfolgt.

19. Verfahren nach einem der Ansprüche 17 und 18, dadurch gekennzeichnet, daß die Umsetzung im Temperaturbereich von −40 bis 100° C, vorzugsweise von −10 bis 50° C, erfolgt.

20. Verfahren nach einem der Ansprüche 17 bis 19, dadurch gekennzeichnet, daß das Halogenid (G) oder Säureanhydrid (H) im äquivalenten Verhältnis oder einem Überschuß mit dem Cyanovinylalkohol (A) umgesetzt wird.

**Claims**

1. Process for the production of $\beta$-alkoxyacrylonitrile of the formula

$$R'O-CH=CR-CN \quad (C)$$

wherein R has the meaning H, straight-chain or branched alkyl residues with 1 to 20 C-atoms, straight-chained or branched residues $-(CH_2)_n-CN$, $-(CH_2)_n-OR''$ or $-(CH_2)_n-CH(OR'')_2$ with n=0 to 5 and R''=alkyl residues with 1 to 12 C-atoms, or $-(CH_2)_{n+1}-Cyc$, with Cyc=isocyclic or heterocyclic, mononuclear or polynuclear aromatic or cycloaliphatic ring systems, which optionally carry substituents on the rings, and n=0 to 5, and wherein R'' has the meaning of a straight-chain or branched alkyl or alkenyl residue with 1 to 12 C-atoms, isocyclic or heterocyclic, mononuclear or polynuclear aromatic or cycloaliphatic ring systems, which optionally carry substituents, or $-(CH_2)_p-Cyc$ with Cyc having the previous meaning, the residues $-(CH_2)_p-OR'''$ or $-(CH_2-CH_2-O)_q-R'''$ with p=1 to 5 and q=1 to 4 and R'''=straight-chained or branched alkyl residues with 1 to 12 C-atoms, by reaction of compounds of the formula

$$\left(\frac{1}{\alpha}Me\right)O-CH=CR-CN \quad (A)$$

wherein R has the above-indicated meaning and Me is an alkali metal with $\alpha=1$ or an alkaline earth metal with $\alpha=2$, with a halogen compound of the formula R'−Hal (B), wherein R' possesses the

above-indicated meaning and Hal denotes chlorine, bromine or iodine, at elevated temperature, characterised in that the reaction takes place in the presence of a basically reacting compound of the alkali or alkaline earth metals.

2. Process according to claim 1, characterised in that a quaternary ammonium salt or a crown ether is added as catalyst.

3. Process according to claim 2, characterised in that there is used as catalyst a quaternary ammonium base of the general formula E

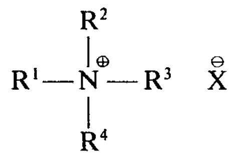 (2)

wherein $R^1$, $R^2$, $R^3$ and $R^4$ denote the same of different cycloalkyl, aryl, alkaryl, aralkyl or alkyl residues, which can be linear or branched, with 1 to 20 C-Atoms, and X denotes a monovalent anion.

4. Process according to claim 2, characterised in that a crown ether is used as catalyst.

5. Process according to one of claims 3 or 4, characterised in that the quaternary ammonium salt or the crown ether is employed in amounts of $10^{-3}$ to $10^{-1}$ mol, related to compound A.

6. Process according to one or more of claims 1 to 5, characterised in that an iodine compound in the form of an iodide or a compound capable of forming iodine ions is added as further catalyst.

7. Process according to claim 6, characterised in that the iodine compound is added in the ratio of $10^{-3}$ to $10^{-1}$ mol per mol of compound of general formula A.

8. Process according to one or more of claims 1 to 7, characterised in that an aprotic medium is used as solvent.

9. Process according to one or more of claims 1 to 8, characterised in that the reaction temperature lies between 60 to 220, preferably between 80 to 180° C.

10. Process for the preparation of 3-aminoacrylonitriles of the formula

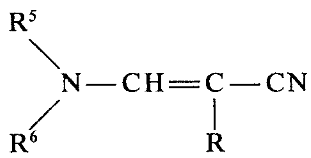 (D)

wherein $R^5$ and $R^6$ have the meaning H, like or different straight-chained or branched alkyl, alkenyl or alkinyl residues with 1 to 12 C-atoms, $-Cyc$, $-(CH_2)_n-Cyc$, wherein Cyc is isocyclic or heterocyclic, mononuclear or polynuclear, aromatic or cycloaliphatic ring systems, which can optionally carry substituents on the rings and $n=0$ to 5, the grouping $-X-R^7$, wherein X is straight-chained, branched or cyclic alkylene or alkenylene residues with 2 to 12 C-atoms, $-Cyc-$, $-(CH_2)_n$-Cyc- or $-(CH_2)_n-Cyc-(CH_2)_n-$ with Cyc and n having the previous meaning and $R^7=$

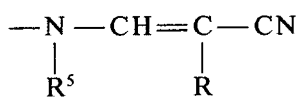

or $R^5$ and $R^6$ together form alkylene or alkenylene residues of a ring with 3 to 6 members, which is optionally interrupted by one or more heteroatoms, and R has the meaning H, straight-chained, branched or cyclic alkyl residues with 1 to 20 C-atoms, straight-chained or branched residues $-(CH_2)_{n+1}-CH$, $-(CH_2)_n-OR''$, $-(CH_2)_n-CH(OR'')_2$ with $R''=$alkyl residues with 1 to 12 C-atoms or $-(CH_2)_{n+1}>Cyc$, wherein $-Cyc$ and n have the above-indicated meaning, wherein however in the case of the residue $-(CH_2)_{n+1}-CN$, n may not be 5, characterised in that a 3-alkoxyacrylonitrile or a metal salt of 3-hydroxyacrylonitrile is reacted with ammonia or a primary or secondary amine, optionally in the presence of a mono- or poly-basic inorganic or organic acid, or with the ammonium salts of these acids.

11. Process for the preparation of 3-ammonoacrylonitriles according to claim 10, of the formula

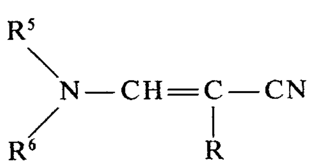 (D)

wherein $R^5$ and $R^6$ have the meaning H, like or different straight-chained or branched alkyl, alkenyl or alkinyl residues with 1 to 12 C-atoms, $-Cyc$, $-(CH_2)_n-Cyc$, wherein Cyc means isocyclic or

heterocyclic, mononuclear or polynuclear, aromatic or cycloaliphatic ring system, which can optionally carry substituents on the rings, and n=0 to 5, the grouping $-X-R^7$, wherein X is straight-chained, branched or cyclic alkylene or alkenylene residues with 2 to 12 C-atoms, —Cyc—, $-(CH_2)_n-Cyc-$ or $-(CH_2)_n-Cyc-(CH_2)_n-$ with Cyc and n having the previous meaning and $R^7=$

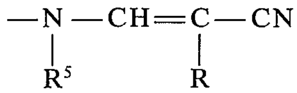

or $R^5$ and $R^6$ together form alkylene or alkenylene residues of a ring with 3 to 6 members, which is optionally interrupted by one or more heteroatoms, and R hás the meaning, straight-chained, branched or cyclic alkyl residues with 1 to 20 C-atoms, straight-chained or branched residues $-(CH_2)_{n+1}-CN$, $-(CH_2)_n$-OR″, $-(CH_2)_n-CH(OR'')_2$ with R″=alkyl residues of 1 to 12 C-atoms or $-(CH_2)_{n+1}-Cyc$, wherein —Cyc and n have the above-indicated meaning, wherein however in the case of the residue $-(CH_2)_{n+1}-CN$, n may not be 5, characterised in that a 3-alkoxyacrylonitrile of the formula

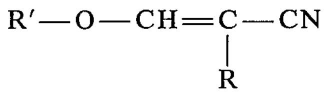
(C)

wherein R has the above-indicated meaning and R′ the meaning of straight-chained or branched alkyl or alkenyl residues of 1 to 12 C-atoms, isocyclic or heterocyclic, mononuclear or polynuclear, aromatic or cycloaliphatic ring systems, which optionally carry substituents, or $-(CH_2)_p-Cyc$ with Cyc having the previous meaning, the residues $-(CH_2)_p-OR'''$ or $-(CH_2-CH_2-O)_q-R'''$ with p=1 to 5 and q=1 to 4 and R‴=straight-chained or branched alkyl residues with 1 to 12 C-Atoms, is reacted with amines of the formula

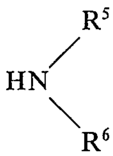
(E)

wherein $R^5$ and $R^6$ have the above-indicated meaning.

12. Process according to claim 11, characterised in that the reaction is carried out at temperatures of −30 to 250° C, preferably from 0° C to the boiling point of the reaction participants.

13. Process according to one of claims 11 or 12, characterised in that the reaction is carried out in the presence of an additional amine which catalyses the reaction.

14. Process for the preparation of a 3-aminoacrylonitrile according to claim 10 of the formula

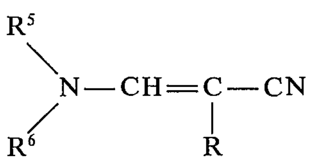
(D)

wherein $R^5$ and $R^6$ have the meaning H, like or different, straight-chained or branched alkyl, alkenyl or alkinyl residues with 1 to 12 C-atoms, —Cyc, $-(CH_2)_n-Cyc$, wherein Cyc is isocyclic or heterocyclic, mononuclear or polynuclear, aromatic or cycloaliphatic ring systems, which can optionally carry substituents on the rings and n=0 to 5, the grouping $-X-R^7$, wherein X is straight-chained, branched or cyclic alkylene or alkenylene residues with 2 to 12 C-atoms, —Cyc—, $-(CH_2)_n-Cyc-$ or $-(CH_2)_n-Cyc-(CH_2)_n$ with Cyc and n having the previous meaning and $R^7=$

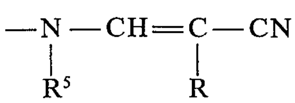

or $R^5$ and $R^6$ together form alkylene or alkenylene residues of a ring with 3 to 6 members, which is optionally interrupted by one or more heteroatoms, and R has the meaning H, straight-chained, branched or cyclic alkyl residues with 1 to 20 C-atoms, straight-chained or branched residues $-(CH_2)_{n+1}-CN$, $-(CH_2)_n-OR'$, $-(CH_2)_n-CH(OR)_2$ with R′=alkyl residues with 1 to 12 C-atoms or $-(CH_2)_{n+1}-Cyc$, wherein —Cyc and n have the above-indicated meaning, but wherein in the case of the residue $-(CH_2)_{n+1}-CN$, n may not be 5, characterised in that a compound of the formula

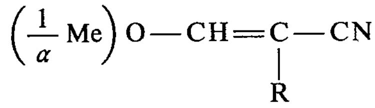

(A)

wherein R has the above-indicated meaning and Me is an alkali metal with $\alpha = 1$ or an alkaline earth metal with $\alpha = 2$ is reacted with an amine of the formula

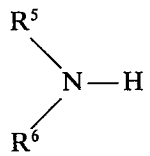

(E)

wherein $R^5$ and $R^6$ have the above-indicated meaning in the presence of a monobasic or polybasic inorganic or organic acid or in the presence of a salt of an inert amine or with an amine salt formed from one of the indicated acids and an amine of formula (E).

15. Process according to claim 14, characterised in that the reaction is carried out in the temperature region of $-10°$ C to 200° C, preferably from 0° C to the boiling point of the reaction participants.

16. Process according to one of claims 14 or 15, characterised in that the ratio of the molar equivalent of the amine of formula (E) or of the amine salts to the substance of formula (A) amounts to 1 : 1 to 6 : 1, preferably 1 : 1 to 3 : 1.

17. Process for the preparation of 2-cyanovinyl esters of the general formula

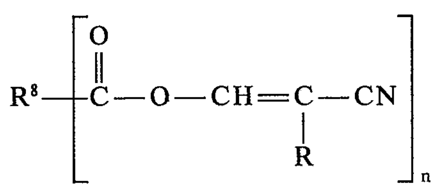

(F)

wherein n is 1 or 2 and $R^8$ denotes straight-chained or branched alkyl, alkylene, alkenyl, or alkenylene residues with 1 to 12 C-atoms, isocyclic or heterocyclic ring systems with mono- or polycyclic structure or $(CH_2)_m-Cyc$, wherein Cyc is an isocyclic or heterocyclic ring with mono- or polycyclic structure, which optionally can carry substituents on the ring and m = 0 to 5, and R denotes H, straight-chained, branched or cyclic alkyl residues with 1 to 20 carbon atoms, straight-chained or branched residues $(CH_2)_p-CN$; $(CH_2)_p-COOR'''$ $(CH_2)_p-NH_2$, $(CH_2)_p-OR'''$, $(CH_2)_{p+1}-Cyc$, wherein Cyc is an isocyclic or heterocyclic ring with mono- or polycyclic structure or an aromatic or heteroaromatic ring with mono- or polycyclic structure, which optionally can carry substituents on the ring, R''' denotes alkyl residues with 1 to 12 C-atoms or residues of monovalent phenols and p = 0 to 5, characterised in that a compound of the formula

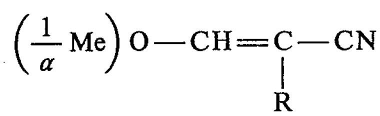

(A)

in which R has the same meaning as in formula (F) and Me is an alkali metal with $\alpha = 1$ or an alkaline earth metal with $\alpha = 2$, is reacted with an acid halide of the formula

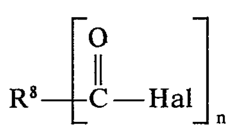

(G)

wherein n and $R^1$ have the meaning given in formula (F) and Hal denotes chlorine or bromine, or an acid anhydride of the formula

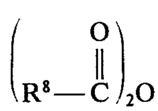

(H)

wherein $R^8$ has the same meaning as in formula (F).

18. Process according to claim 17, characterised in that the reaction takes place in an inert, polar or non-polar organic solvent.

19. Process according to one of claims 17 and 18, characterised in that the reaction takes place in the temperature region of −40 to 100° C, preferably from −10 to 50° C.

20. Process according to one of claims 17 to 19, characterised in that the halide (G) or acid anhydride (H) is reacted in the equivalent ration or an excess with respect to the cyanovinyl alcohol (A).

## Revendications

1. Procédé pour la préparation de $\beta$-alcoxyacrylonitriles de formule:

$$R'O-CH=CR-CN \qquad (C)$$

dans laquelle R représente l'hydrogène, des radicaux alkyle, à chaîne droite ou ramifiée, ayant de l à 20 atomes de carbone, des radicaux $-(CH_2)_n-CN$, $-(CH_2)_n-OR''$ ou $-(CH_2)_n-CH(OR'')_2$, à chaîne droite ou ramifiée, où n = de 0 à 5 et R'' représente des radicaux alkyle ayant de 1 à 12 atomes de carbone, ou des radicaux $-(CH_2)_{n+1}-Cyc$, avec Cyc représentant des systèmes isocycliques ou hétérocycliques, aromatiques ou cycloaliphatiques à un seul ou à plusieurs cycles, qui portent éventuellement des substituants sur les cycles, et n = de 0 à 5 et où R' représente des radicaux alkyle ou alcényle, à chaîne droite ou ramifiée, ayant de 1 à 12 atomes de carbone, des systèmes isocycliques ou hétérocycliques, aromatiques ou cycloaliphatiques à un ou plusieurs cycles qui portent éventuellement des substituants, ou $-(CH_2)_p-Cyc$ où Cyc a la signification précédente, des radicaux $-(CH_2)_p-OR'''$ ou $-(CH_2-CH_2-O)_q-R'''$ avec p = de 1à 5 et q = de 1 à 4 et R''' représentant des radicaux alkyle, à chaîne droite ou ramifiée, ayant de 1 à 12 atomes de carbone, par réaction à température élevée de composés de formule:

$$\left(\frac{1}{\alpha}\,Me\right)O-CH=CR-CN \qquad (A)$$

dans laquelle R a la signification indiquée ci-dessus et Me est un métal alcalin avec $\alpha = 1$ ou un métal alcalinoterreux avec $\alpha = 2$, avec un composé halogéné de formule R'—Hal (B) où R' a la signification indiquée ci-dessus et Hal désigne le chlore, le brome ou l'iode, lequel procédé est caractérisé en ce que la réaction a lieu en présence d'un composé à réaction basique de métaux alcalins ou alcalinoterreux.

2. Procédé selon la revendication 1, caractérisé en ce qu'on ajoute un sel d'ammonium quaternaire ou un éther-couronne à titre de catalyseur.

3. Procédé selon la revendication 2, caractérisé en ce qu'on utilise à titre de catalyseur une base ammonium quaternaire de formule générale (2):

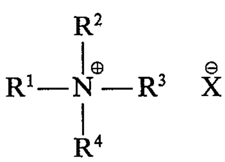

(2)

dans laquelle $R_1$, $R_2$, $R_3$ et $R_4$ représent des radicaux cycloalkyle, aryle, alkylaryle, arylalkyle ou alkyle identiques ou différents, qui peuvent être linéaires ou ramifiés, ayant de 1 à 20 atomes de carbone et X est un anion monovalent.

4. Procédé selon la recendication 2, caractérisé en ce qu'à titre de catalyseur on utilise un éther-couronne.

5. Procédé selon l'une des revendications 3 ou 4, caractérisé en ce que le sel d'ammonium quaternaire ou l'éther-couronne est utilisé en des quantités de $10^{-3}$ à $10^{-1}$ mole par mole du composé (A).

6. Procédé selon l'une ou plusieurs des revendications 1 à 5, caractérisé en ce que comme autre catalyseur on ajoute un composé d'iode sous la forme d'un iodure ou d'un composé capable de former des ions d'iode.

7. Procédé selon la revendication 6, caractérisé en ce que le composé d'iode est ajouté en une proportion allant de $10^{-3}$ à $10^{-1}$ mole par mole du composé de formule générale A.

8. Procédé selon l'une ou plusieurs des revendications 1 à 7, caractérisé en ce qu'on utilise un milieu aprotique comme solvant.

9. Procédé selon l'une ou plusieurs des revendications 1 à 8, caractérisé en ce que la température de réaction est comprise entre 60 et 220° C de préférence entre 80 et 180° C.

10. Procédé pour la préparation de 3-aminoacrylonitriles de formule:

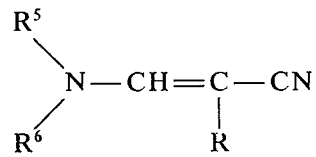

(D)

dans laquelle $R^5$ et $R^6$ représentent l'hydrogène ou des radicaux alkyle, alcényle ou alcynyle, identiques ou différents, à chaîne droite ou ramifiée, ayant de 1 à 12 atomes de carbone, des radicaux $-Cyc$, $-(CH_2)_n-Cyc$ où Cyc désigne des systèmes isocycliques, ou hétérocycliques, aromatiques ou cycloaliphatiques à un ou plusieurs cycles, qui peuvent porter éventuellement des substituants sur les cycles et n = de 0 à 5, le groupe $-X-R^7$ où X représente des radicaux alkylène ou alcénylène, à chaîne droite ou ramifiée, ou cycliques ayant de 2 à 12 atomes de carbone, des radicaux $-Cyc-$, $-(CH_2)_n-Cyc-$ ou $(CH_2)_n-Cyc-(CH_2)_n$ où Cyc et n ont les significations ci-dessus et $R^7$ =

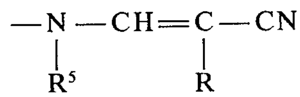

où $R_5$ et $R_6$ forment ensemble des radicaux alkylène ou alcénylène d'un cycle ayant de 3 à 6 membres, qui peut être éventuellement interrompu par un ou plusieurs hétéroatomes, et R représente l'hydrogène, des radicaux alkyle, à chaîne droite ou ramifiée, ou cycliques, ayant de 1 à 20 atomes de carbone, des radicaux $-(CH_2)_{n+1}-CN$, $-(CH_2)_nOR''$, $-(CH_2)_n-CH(OR'')_2$ à chaîne droite ou ramifiée, où R″ désigne des radicaux alkyle ayant de 1 à 12 atomes de carbone, ou $-(CH_2)_{n+1}-Cyc$ où Cyc et n ont les significations indiquées ci-dessus, mais auquel cas lorsque le radical est $-(CH_2)_{n+1}-CN$, n ne doit pas être 5, lequel procédé est caractérisé en ce qu'on fait réagir un 3-alcoxyacrylonitrile ou un sel métallique de 3-hydroxyacrylonitriles avec de l'ammoniac ou une amine primaire ou secondaire, éventuellement en présence d'un mono- ou poly- acide minéral ou organique, avec des sels d'ammonium de ces acides.

11. Procédés pour la préparation de 3-aminoacrylonitriles selon la revendication 10, de formule:

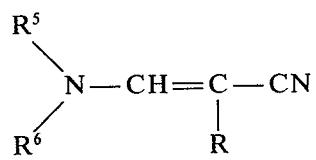

(D)

dans laquelle $R^5$ et $R^6$ représentent l'hydrogène, des radicaux alkyle, alcényle ou alcynyle, à chaîne droite ou ramifiée, ayant de I à 12 atomes de carbone, identiques ou différents, des groupes $-Cyc$, $-(CH_2)_n-Cyc$, où Cyc représente un système isocyclique ou hétérocyclique, aromatique ou cycloaliphatique à un ou plusieurs cycles, qui peut éventuellement porter des substituants sur les cycles et n = de 0 à 5, le groupe $-X-R^7$ où X représente des radicaux alkylène ou alcénylène à chaîne droite ou ramifiée, ou cycliques ayant de 2 à 12 atomes de carbone, des radicaux $-Cyc-$, $-(CH_2)_n-Cyc-$ ou $-(CH_2)_n-Cyc-(CH_2)_n-$ où Cyc et n ont les mêmes significations que ci-dessus et $R^7$ représente

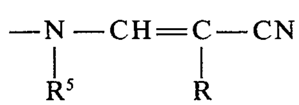

où $R^5$ et $R^6$ forment ensemble des radicaux alkylène ou alcénylène d'un cycle ayant de 3 à 6 membres, qui peut éventuellement être interrompu par un ou plusieurs hétéroatomes, et R est l'hydrogene, des radicaux alkyle à chaîne droite ou ramifiée, ou cycliques, ayant de 1 à 20 atomes de carbone, ou des radicaux à chaîne droite ou ramifiée de formule $-(CH_2)_{n+1}-CN$, $-(CH_2)_nOR''$, $-(CH_2)_n-CH(OR'')_2$ où R″ désigne des radicaux alkyle ayant de 1 à 12 atomes de carbone, ou $-(CH_2)_{n+1}-Cyc$ où $-Cyc$ et n ont les significations indiquées ci-dessus, mais auquel cas lorsque le radical est $-(CH_2)_{n+1}-CN$ n ne doit pas être 5, lequel procédé est caractérisé en ce qu'on fait réagir un 3-alcoxyacrylonitrile de formule:

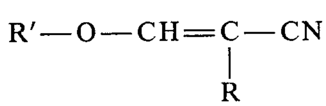

(C)

dans laquelle R a la signification indiquée ci-dessus et R′ représente des radicaux alkyle ou alcényle à chaîne droite ou ramifiée ayant de 1 à 12 atomes de carbone, des systèmes isocycliques ou

hétérocycliques, aromatiques ou cycloaliphatiques à un ou plusiers cycles, qui peuvent éventuellement porter des substituants, ou $-(CH_2)_n-Cyc$, Cyc ayant la signification ci-dessus, les radicaux $-(CH_2)_p-OR'''$ ou $-(CH_2-CH_2-O)_q-R'''$ où p = de 1 à 5 et q = de 1 à 4 et R''' représente des radicaux alkyle, à chaîne droite ou ramifiée, ayant de 1 à 12 atomes de carbone, avec des amines de formule

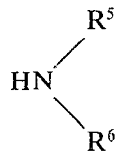

(E)

dans laquelle $R^5$ et $R^6$ ont les significations ci-dessus.

12. Procédé selon la revendication 11, caractérisé en ce que la réaction est conduite entre −30 et 250° C, de préference entre 0° C et le point d'ébullition des réactifs.

13. Procédé selon l'une des revendications 11 ou 12, caractérisé en ce que la réaction est conduite en présence d'une amine supplémentaire qui catalyse la réaction.

14. Procédé pour la préparation de 3-aminoacrylonitriles selon la revendication 10, de formule

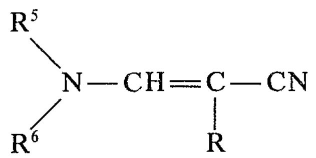

(D)

dans laquelle $R^5$ et $R^6$ représentent l'hydrogène, des radicaux alkyle, alcényle ou alcynyle, à chaîne droite ou ramifiée, ayant de 1 à 12 atomes de carbone, identiques ou différents, des radicaux −Cyc, $-(CH_2)_n-Cyc$ où Cyc désigne des systèmes isocycliques, ou hétérocycliques, aromatiques ou cycloaliphatiques à un ou plusieurs cycles, qui peuvent éventuellement porter des substituants sur les cycles et n = de 0 à 5, le groupe $-X-R^7$ où X désigne des radicaux alkylène ou alcénylène ayant de 2 à 12 atomes de carbone, −Cyc, $-(CH_2)_n-Cyc-$ ou $-(CH_2)_n-Cyc-(CH_2)_n-$ où Cyc et n ont les significations ci-dessus et $R^7$ est

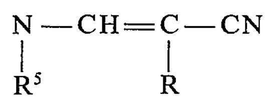

ou $R^5$ et $R^6$ forment ensemble des radicaux alkylène ou alcénylène d'un cycle ayant de 3 à 6 membres qui est éventuellement interrompu par un ou plusieurs hétéroatomes, et R représente l'hydrogène, des radicaux alkyle, à chaîne droite ou ramifiée, ou cycliques ayant de 1 à 20 atomes de carbone, des radicaux $-(CH_2)_{n+1}-CN$, $-(CH_2)_n-OR'$, $-(CH_2)_n-CH(OR)_2$ à chaîne droite ou ramifiée, où R' désigne des radicaux alkyle ayant de 1 à 12 atomes de carbone ou $(CH_2)_{n+1}-Cyc$ où Cyc et n ont les significations ci-dessus, mais auquel cas lorsque le radical est $-(CH_2)_{n+1}-CN$, n de doit pas être 5, lequel procédé est caractérisé en ce qu'on fait réagir un composé de formule

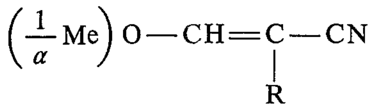

(A)

dans laquelle R a la signification ci-dessus et Me est un métal alcalin avec $\alpha = 1$ ou un métal alcalino-terreux avec $\alpha = 2$, avec une amine de formule

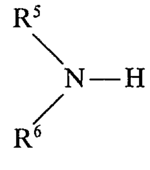

(E)

dans laquelle $R^5$ et $R^6$ ont la signification indiquée ci-dessus, en présence d'un mono- ou poly- acide minéral ou organique ou en présence d'un sel d'une amine inerte ou avec un sel d'amine d'un des acides mentionnés et d'une amine de formule (E).

15. Procédé selon la revendication 14, caractérisé en ce que la réaction est conduite à une température comprise entre −10 et 200° C, de préference entre 0° C et la température d'ébullition des réactifs.

16. Procédé selon l'une des revendications 14 ou 15, caractérisé en ce que le rapport des équivalents molaires de l'amine de formule (E) ou du sel d'amine à la substance de formule (A) est de 1 : 1 à 6 : 1, de préférence de 1 : 1 à 3 : 1.

17. Procédé pour la préparation d'esters de 2-cyanovinyle de formule générale

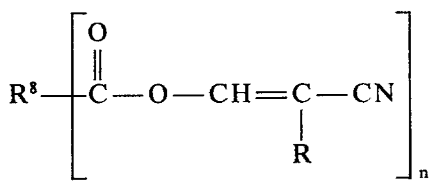

(F)

dans laquelle n est 1 ou 2 et $R^8$ représente des radicaux alkyle, alkylène, alcényle ou alcénylène à chaîne droite ou ramifiée, ayant de 1 à 12 atomes de carbone, des systèmes isocycliques ou hétérocycliques avec une structure à un ou plusieurs cycles ou $(CH_2)_m-Cyc$, où Cyc est un noyau isocyclique ou hétérocyclique avec une structure à un ou plusieurs cycles, qui peut éventuellement porter des substituants sur les cycles et m = de 0 à 5, et R représente l'hydrogène, des radicaux alkyle à chaîne droite ou ramifiée, ou cycliques, ayant de 1à 20 atomes des carbone, des radicaux $-(CH_2)_p-CN$, $-(CH_2)_p-COOR'''$, $-CH_2)_p-NH_2$, $-(CH_2)_p-OR'''$, $-(CH_2)_{p+1}-Cyc$ à chaîne droite ou ramifiée où Cyc désigne un système isocyclique ou hétérocyclique avec une structure à un ou plusieurs cycles ou un cycle aromatique ou hétéroaromatique avec une structure à un ou plusieurs cycles, qui peut éventuellement porter des substituants sur les cycles, R''' représente des radicaux alkyle ayant de 1 à 12 atomes de carbone ou des restes de phénols monovalents et p = de 0 à 5, lequel procédé est caractérisé en ce qu'on fait réagir un composé de formule:

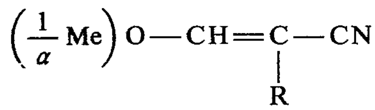

(A)

dans laquelle R a la même signification que dans la formule (F) et Me est un métal alcalin avec $\alpha = 1$ ou un métal alcalino-terreux avec $\alpha = 2$, avec un halogénure d'acide de formule

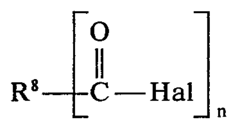

(G)

dans laquelle n et $R^1$ ont les significations indiquées pour la formule (F) et Hal désigne le chlore ou le brome, ou avec un anhydride d'acide de formule

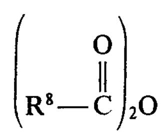

(H)

dans laquelle $R^8$ a la même signification que dans la formule (F).

18. Procédé selon la revendication 17, caractérisé en ce que la réaction a lieu dans un solvant organique, inerte, polaire ou non polaire.

19. Procédé selon l'une des revendications 17 et 18, caractérisé en ce que la réaction est conduite à une température comprise entre − 40 et 100° C, de préférence entre − 10 et 50° C.

20. Procédé selon l'une quelconque des revendications 17 à 19, caractérisé en ce que l'halogénure (G) ou l'anhydride d'acide (H) est amené à réagir avec l'alcool cyanovinylique (A) dans un rapport équivalent ou en excès.